(19) Europäisches Patentamt
European Patent Office
Office européen des brevets

(11) EP 3 774 716 B1

# (12) EUROPÄISCHE PATENTSCHRIFT

(45) Veröffentlichungstag und Bekanntmachung des Hinweises auf die Patenterteilung:
**16.03.2022 Patentblatt 2022/11**

(21) Anmeldenummer: **19712230.2**

(22) Anmeldetag: **27.03.2019**

(51) Internationale Patentklassifikation (IPC):
***C07C 209/16*** (2006.01) ***C07C 211/18*** (2006.01)
***C07C 213/02*** (2006.01) ***C07C 215/20*** (2006.01)

(52) Gemeinsame Patentklassifikation (CPC):
(C-Sets verfügbar)
**C07C 209/16; C07C 211/18; C07C 213/02; C07C 215/20** (Forts.)

(86) Internationale Anmeldenummer:
**PCT/EP2019/057682**

(87) Internationale Veröffentlichungsnummer:
**WO 2019/192903 (10.10.2019 Gazette 2019/41)**

## (54) VERFAHREN ZUR HERSTELLUNG VON AMINEN

PROCESS FOR THE PREPARATION OF AMINES

PROCEDE DE PREPARATION D'AMINES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **06.04.2018 EP 18165995**

(43) Veröffentlichungstag der Anmeldung:
**17.02.2021 Patentblatt 2021/07**

(73) Patentinhaber: **BASF SE**
**67056 Ludwigshafen am Rhein (DE)**

(72) Erfinder:
- **ERNST, Martin**
  **67056 Ludwigshafen (DE)**
- **ALTENHOFF, Ansgar Gereon**
  **67056 Ludwigshafen (DE)**
- **LUYKEN, Hermann**
  **67056 Ludwigshafen (DE)**

(74) Vertreter: **BASF IP Association**
**BASF SE**
**GBI-C006**
**67056 Ludwigshafen (DE)**

(56) Entgegenhaltungen:
**WO-A1-2016/023837 WO-A1-2017/037069**

EP 3 774 716 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

Printed by Jouve, 75001 PARIS (FR)

(Forts. nächste Seite)

(52) Gemeinsame Patentklassifikation (CPC): (Forts.)

C-Sets
**C07C 209/16, C07C 211/18;**
**C07C 213/02, C07C 215/20**

## Beschreibung

Die vorliegende Erfindung betrifft ein Verfahren zur Herstellung von Aminen der Formel (1),

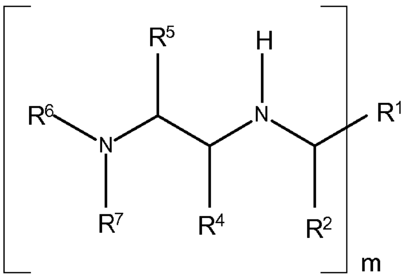

(1)

in der

m für 1, 2 oder 3 steht, wobei

wenn m für 1 steht

$R^1$ aliphatischer $C_{1-60}$-Kohlenwasserstoffrest oder $C_{4-60}$-Kohlenwasserstoffrest, der mindestens einen cycloaliphatischen oder aromatischen Ring enthält, bedeutet, wobei ein solcher $C_{1-60}$- oder $C_{4-60}$-Kohlenwasserstoffrest gegebenenfalls ein oder mehrere Heteroatome, unabhängig voneinander ausgewählt aus der Gruppe bestehend aus Stickstoff (N), Sauerstoff (O), und Schwefel (S) enthalten kann, und
$R^2$ Wasserstoff (H), $C_{1-6}$-Alkyl oder Phenyl bedeutet,
oder
$R^1$, $R^2$ gemeinsam $-(CH_2)_j-Y-(CH_2)_k-$ bedeuten, wobei Y Methylen, Sauerstoff (O), Schwefel (S), oder $NR^3$ (wobei $R^3$ $C_{1-4}$-Alkyl ist) bedeutet und j und k unabhängig voneinander eine ganze Zahl von 1 bis 4 bedeuten,

wenn m für 2 oder 3 steht

$R^1$ zwei- oder dreiwertiger $C_{4-20}$-Kohlenwasserstoffrest bedeutet, der mindestens einen cycloaliphatischen oder aromatischen Ring enthält, wobei ein solcher Kohlenwasserstoffrest gegebenenfalls ein oder mehrere Heteroatome, unabhängig voneinander ausgewählt aus der Gruppe bestehend aus Stickstoff (N), Sauerstoff (O), und Schwefel (S) enthalten kann, und

$R^2$ Wasserstoff (H), $C_{1-6}$-Alkyl oder Phenyl bedeutet, und

$R^4$ und $R^5$ unabhängig voneinander Wasserstoff (H) oder $C_{1-16}$-Alkyl bedeuten,

$R^6$ und $R^7$ unabhängig voneinander Wasserstoff (H), oder $C_{1-4}$-Alkyl bedeuten.

STAND DER TECHNIK

Solche Amine finden u.a. Verwendung als Zwischenprodukte bei der Herstellung von biologisch aktiven Substanzen (Mokrov G. V. et al, Russian Chemical Bulletin, 59(6), 1254-1266, 210). Ebenso finden sie Verwendung als Vernetzer in Polyurethanschäumen (US 8,552,078 B2). Wege zur Herstellung solcher Amine sind allgemein bekannt.
Mokrov G. V. et al (Mokrov G. V. et al, Russian Chemical Bulletin, 59(6), 1254-1266, 210) beschreibt u.a. die Herstellung von N-Benzyl-1,2-Ethandiamin durch Umsetzung von Benzaldehyd mit einem 3-fachen Überschuss an 1,2-Ethandiamin (1,2-EDA).
Kurganov A. et al (Liebigs Ann. Chem. 1980, 786 - 790) beschreibt u.a. die Herstellung von $N^1$-Benzyl-1,2-propandiamin durch Umsetzung von Benzaldehyd mit 1,2-Propandiamin (1,2-PDA).
US 8,552,078 B2 (Air Products and Chemicals, Inc.) beschreibt die Umsetzung von Polyaminen mit geeigneten Aldehyden und Ketonen, bspw. die Umsetzung von 1,2-EDA mit Benzaldehyd zum N-Benzyl-1,2-ethylendiamin.
WO 2016/023839 A1 (Sika Technology AG) beschreibt die Umsetzung von 1,2-PDA mit einem entsprechenden Aldehyd oder Keton (bspw. die Umsetzung mit Benzaldehyd zum $N^1$-Benzyl-1,2-propylendiamin).
WO 2017/037069 A1 (Sika Technology AG) beschreibt die Umsetzung von 1,2-EDA mit einem entsprechenden Aldehyd oder Keton (bspw. die Umsetzung mit Benzaldehyd zum N-Benzyl-1,2-ethandiamin). Als Nebenprodukt tritt

hauptsächlich mehrfach alkyliertes 1,2-EDA (bspw. N,N'-Benzyl-1,2-Ethylendiamin) auf.

WO 2016/023837 A1 (Sika Technology AG) beschreibt die Umsetzung von 1,2-PDA mit einer di- oder trifunktionellen Carbonylverbindung (bspw. die Umsetzung mit Terephthalaldehyd zu 1,4-Bis(2-aminopropylaminomethyl)benzol).

WO 2017/037070 A1 (Sika Technology AG) beschreibt die Umsetzung von 1,2-EDA mit einer di- oder trifunktionellen Carbonylverbindung (bspw. die Umsetzung mit Terephthalaldehyd zu 1,4-Bis(2-aminoethylaminomethyl)benzol).

Gemäß dem oben beschriebenen Stand der Technik lässt sich ein entsprechend alkyliertes Amin durch Umsetzung von 1,2-EDA oder 1,2-PDA mit einem entsprechenden Aldehyd oder Keton herstellen. Dabei werden unerwünschte Nebenprodukte gebildet, in denen beide Stickstoffatome alkyliert werden. Wird 1,2-EDA/1,2-PDA mit einem Aldehyd/Keton äquimolar umgesetzt, so ist die Selektivität verbesserungswürdig, da entsprechende Nebenprodukte in nicht unerheblichen Mengen gebildet werden. Die Bildung solcher Nebenprodukte lässt sich verringern, indem ein Überschuss von 1,2-EDA bzw. 1,2-PDA eingesetzt wird. Hieraus ergibt sich aber die Problematik, dass das überschüssige Diamin vom Reaktionsgemisch abgetrennt werden muss, was vornehmlich mittels Destillation erfolgt. D.h. durch den Einsatz eines Überschusses an Diamin (Edukt) lässt sich zwar die Selektivität steigern, gleichzeitig steigen aber auch die laufenden Kosten des Verfahrens wegen des Energieaufwands, der für die Destillation erforderlich ist.

Insbesondere, wenn kleinere Mengen eines gewünschten Amins hergestellt werden sollen, (bspw. einige wenige Tonnen), ergibt sich ein weiteres Problem. Das destillativ abgetrennte Diamin (Edukt) kann nicht beliebig in die Reaktion zurückgeführt werden. D.h. nach Herstellung der gewünschten Menge des Amins muss das abgetrennte Diamin (Edukt) üblicherweise entsorgt (bspw. verbrannt) werden. Dies stellt insbesondere bei vergleichsweise teuren Edukten wie 1,2-EDA oder 1,2-PDA einen erheblichen wirtschaftlichen Nachteil dar.

Der vorliegenden Erfindung lag daher die Aufgaben zugrunde, die Wirtschaftlichkeit bisheriger Verfahren zur Herstellung von Aminen der Formel (1) zu verbessern und einem Nachteil oder mehreren Nachteilen des Standes der Technik, insbesondere den o.g. Nachteilen, abzuhelfen. Es sollte also ein Verfahren gefunden werden, dass es erlaubt, Amine der Formel (1) mit hohem Umsatz, hoher Ausbeute, Raum-Zeit-Ausbeute (RZA), und Selektivität herzustellen. Ein solches Verfahren sollte es ferner erlauben, Amine der Formel (1) auf Basis anderer Ausgangsmaterialen als entsprechender Diamine (beispielsweise 1,2-EDA oder 1,2-PDA) herzustellen.

[Raum-Zeit-Ausbeuten werden angegeben in ‚Produktmenge / (Katalysatorvolumen • Zeit)' (kg/($l_{Kat.}$ • h)) und/oder ‚Produktmenge / (Reaktorvolumen • Zeit)' (kg/($l_{Reaktor}$ • h)].

Überraschender Weise wurde ein Verfahren zur Herstellung eines Amins der Formel (1)

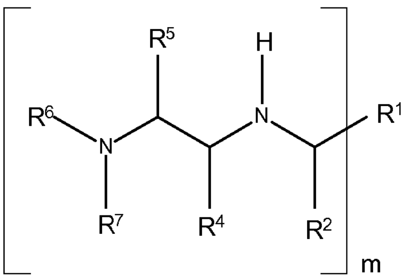

(1)

gefunden, in der

m für 1, 2 oder 3 steht, wobei

wenn m für 1 steht

$R^1$ aliphatischer $C_{1-60}$-Kohlenwasserstoffrest oder $C_{4-60}$-Kohlenwasserstoffrest, der mindestens einen cycloaliphatischen oder aromatischen Ring enthält, bedeutet, wobei ein solcher $C_{1-60}$- oder $C_{4-60}$-Kohlenwasserstoffrest gegebenenfalls ein oder mehrere Heteroatome, unabhängig voneinander ausgewählt aus der Gruppe bestehend aus Stickstoff (N), Sauerstoff (O), und Schwefel (S) enthalten kann, und
$R^2$ Wasserstoff (H), $C_{1-6}$-Alkyl oder Phenyl bedeutet,
oder
$R^1$, $R^2$ gemeinsam $-(CH_2)_j-Y-(CH_2)_k-$ bedeuten, wobei Y Methylen, Sauerstoff (O), Schwefel (S), oder $NR^3$ (wobei $R^3$ $C_{1-4}$-Alkyl ist) bedeutet und j und k unabhängig voneinander eine ganze Zahl von 1 bis 4 bedeuten,

wenn m für 2 oder 3 steht

$R^1$ zwei- oder dreiwertiger $C_{4-20}$-Kohlenwasserstoffrest bedeutet, der mindestens einen cycloaliphatischen oder aromatischen Ring enthält, wobei ein solcher Kohlenwasserstoffrest gegebenenfalls ein oder mehrere Heteroatome, unabhängig voneinander ausgewählt aus der Gruppe bestehend aus Stickstoff (N), Sauerstoff (O), und Schwefel (S) enthalten kann, und

$R^2$ Wasserstoff (H), $C_{1-6}$-Alkyl oder Phenyl bedeutet, und

$R^4$ und $R^5$ unabhängig voneinander Wasserstoff (H) oder $C_{1-16}$-Alkyl bedeuten,

$R^6$ und $R^7$ unabhängig voneinander Wasserstoff (H), oder $C_{1-4}$-Alkyl bedeuten,

umfassend die Schritte

1. Umsetzung eines Aminoalkohols der Formel 2

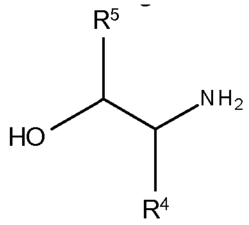

(2)

mit einer Carbonylverbindung der Formel (3)

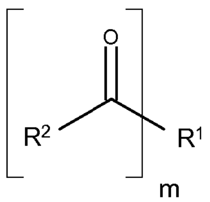

(3)

und anschließender Hydrierung des resultierenden Reaktionsproduktes mit Wasserstoff ($H_2$) an einem Heterogen-Hydrierkatalysator zu einem Intermediat der Formel (4)

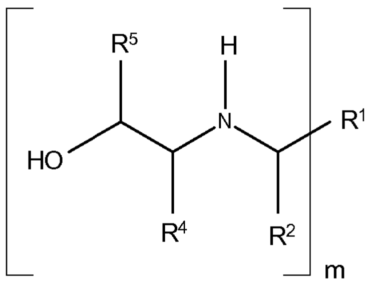

(4)

2. Umsetzung des in Schritt 1 erhaltenen Intermediats mit einer Aminkomponente der Formel (5)

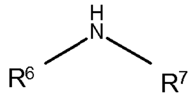

(5)

in Gegenwart von Wasserstoff ($H_2$) und eines Heterogen-Hydrierkatalysators zu einem entsprechenden Amin der Formel (1),

wobei m sowie die Reste $R^1$, $R^2$, $R^4$, $R^5$, $R^6$ und $R^7$ in den Formeln (2) bis (5) dieselbe Bedeutung wie in Formel (1) haben.

Es wurde überraschenderweise gefunden, dass man mithilfe des erfindungsgemäßen Verfahrens Amine der Formel (1) mit einer hohen Ausbeute und Selektivität erhält. Grundsätzlich hätte der Fachmann erwartet, dass in der ersten als auch in der zweiten Stufe Ausbeuteverluste auftreten, die insgesamt größer sind, als die des einstufigen Verfahrens gemäß Stand der Technik. Entscheidend für diese Erwartung ist, dass es sich bei der zweiten Stufe (Schritt 2) um eine Alkoholaminierung handelt, die verglichen mit der Aminierung eines Ketons oder Aldehyds üblicherweise bei höheren Temperaturen und Drücken stattfindet. Die höheren Temperaturen und Drücke sind erforderlich, um die entsprechende Alkoholgruppe zunächst zum Aldehyd oder Keton zu oxidieren (dehydrieren), welches dann mit der Aminkomponente reagieren kann. Üblicherweise steigt mit zunehmender Temperatur und zunehmendem Druck aber auch die Wahrscheinlichkeit, dass unerwünschte Nebenreaktionen in signifikanter Anzahl stattfinden. Insofern würde der Fachmann die Aminierung eines Aldehyds oder Ketons der eines Alkohols vorziehen. Insbesondere würde er nicht erwarten, dass die Selektivität des Verfahrens des Standes der Technik (eine Stufe, Aminierung eines Aldehyds bzw. Ketons) geringer als die des erfindungsgemäßen Verfahrens ist (zwei Stufen; 1. Stufe: Aminierung eines Aldehyds bzw. Ketons, 2. Stufe Aminierung eines Alkohols).

BESCHREIBUNG DER ERFINDUNG

Mithilfe des erfindungsgemäßen Verfahrens lassen sich Amine der Formel (1) mit hoher Ausbeute und Selektivität herstellen, wobei als Edukte Aminoalkohole der Formel (2), beispielsweise 1-Aminopropan-2-ol (MIPOA) oder Monoethanolamin (MEOA), eingesetzt werden. Solche Edukte lassen sich einfacher und damit kostengünstiger herstellen, als die im Stand der Technik eingesetzten Diamine wie 1,2-EDA oder 1,2-PDA.

Beschreibung der Reste $R^1$ bis $R^7$:

Allgemeines:

Als "aliphatischer Rest" wird ein Rest bezeichnet, der weder einen cycloaliphatischen noch einen aromatischen Ring enthält. Er kann linear oder verzweigt sein. Erfindungsgemäß kann das lineare oder verzweigte Kohlenstoffgrundgerüst ferner entsprechende Heteroatome, also Stickstoff (N), Sauerstoff (O) und Schwefel (S) enthalten.

Steht m für 1 ist $R^1$ ein einwertiger, steht m für 2 ist $R^1$ ein zweiwertiger und steht m für 3 ist $R^1$ ein dreiwertiger Rest. Die Wertigkeit von $R^1$ entspricht folglich dem Wert von m.

Bevorzugt ist $R^1$ gesättigt. Im Falle eines Kohlenwasserstoffrestes der mindestens einen aromatischen Ring enthält ist hierunter zu verstehen, dass $R^1$ keine CC-Doppelbindungen enthält, außer denen, die formal Bestandteil des aromatischen Rings sind.

Enthält $R^1$ Heteroatome, so sind diese bevorzugt wie folgt in den Kohlenwasserstoffrest eingebaut: C-O-C, C-S-C, C-N=C, C-N-CC, besonders bevorzugt C-O-C, C-S-C, C-N-CC, ganz besonders bevorzugt C-O-C, C-N-CC oder sogar C-O-C (C-N-CC bedeutet, dass der Stickstoff mit allen drei Kohlenstoffatomen verbunden ist).

Die erfindungsgemäßen Heteroatome können sowohl Bestandteil des cycloaliphatischen oder aromatischen Rings sein, als auch außerhalb des Rings liegen. Furyl ist ein Beispiel für einen Rest, in dem das Heteroatom Bestandteil des aromatischen Rings ist. Entsprechend ist im 2,5-Tetrahydrophenylen das Heteroatom Bestandteil des cycloaliphatischen Rings. 4-Methoxybenzyl ist ein Beispiel für einen Rest, in dem das Heteroatom außerhalb des aromatischen Rings liegt. In allen Fällen ist der Sauerstoff als C-O-C in das Kohlenstoffgrundgerüst eingebaut.

Ein Alkoxyalkylrest ist ein Beispiel für einen aliphatischen Rest, der ein entsprechendes Heteroatom (Sauerstoff (O)) enthält

Eine gestrichelte Linie in den erfindungsgemäßen Formeln stellt jeweils die Bindung zwischen dem Rest $R^1$ und dem zugehörigen Molekülrest (d.h. dem sich in den Formeln (1), (3) und (4) in eckigen Klammern befindenden Molekülfragment) dar.

m kann 1,2 oder 3 bedeuten. Bevorzugt bedeutet m 1 oder 2, besonders bevorzugt 1.

Herstellung von Aminen in denen m für 1 steht:

Das erfindungsgemäßen Verfahren wird im Folgenden zunächst im Hinblick auf die Herstellung solcher Amine näher beschrieben, in denen m für 1 steht. Bevorzugt ist dabei die Herstellung solcher Amine in denen die Reste $R^1$ bis $R^7$ die im Folgenden beschriebenen Bedeutungen aufweisen.

Das erfindungsgemäße Verfahren eignet sich insbesondere zur Herstellung von Aminen der Formel (1) in der m für 1 steht, wobei die Reste $R^1$, $R^2$, $R^6$ und $R^7$ nicht so gewählt sind, dass die Gruppe $R^6R^7N$- der Gruppe $-NHCHR^1R^2$

entspricht. Beispielsweise soll $R^6$ nicht Ethyl und $R^7$ Wasserstoff (H) sein und gleichzeitig $R^1$ Methyl und $R^2$ Wasserstoff (H) bedeuten.

<u>$R^1$ und $R^2$:</u>

Bevorzugt ist $R^1$ ein aliphatischer $C_{5-50}$-, besonders bevorzugt $C_{6-40}$- und ganz besonders bevorzugt $C_{7-30}$- oder sogar $C_{8-20}$-Kohlenwasserstoffrest.

Ebenfalls bevorzugt ist $R^1$ ein $C_{4-60}$-, besonders bevorzugt $C_{5-50}$- und ganz besonders bevorzugt $C_{6-40}$- oder sogar $C_{7-30}$-Kohlenwasserstoffrest, der mindestens einen cycloaliphatischen oder aromatischen Ring enthält.

$R^1$ kann beispielsweise

- $C_{3-50}$-Alkyl (bevorzugt $C_{4-40}$-Alkyl, besonders bevorzugt $C_{5-30}$-Alkyl, ganz besonders bevorzugt $C_{6-20}$-Alkyl),
- $C_{3-50}$-Alkoxyalkyl (bevorzugt $C_{4-40}$- Alkoxyalkyl, besonders bevorzugt $C_{5-30}$- Alkoxyalkyl, ganz besonders bevorzugt $C_{6-20}$- Alkoxyalkyl),
- $C_{4-12}$-Cycloalkyl (bevorzugt $C_{4-10}$-Cycloalkyl, besonders bevorzugt $C_{5-8}$-Cycloalkyl, ganz besonders bevorzugt Cyclohexyl),
- $C_{4-30}$-Alkoxycycloalkyl (bevorzugt $C_{6-20}$-Alkoxycycloalkyl, besonders bevorzugt $C_{7-15}$-Alkoxycycloalkyl, ganz besonders bevorzugt $C_{8-12}$-Alkoxycycloalkyl),
- $C_{4-30}$-Alkylcycloalkyl (bevorzugt $C_{6-20}$-Alkylcycloalkyl, besonders bevorzugt $C_{7-15}$-Alkylcycloalkyl, ganz besonders bevorzugt $C_{8-12}$-Alkoylcycloalkyl), oder
- Rest der Formel (A), (B), oder (C) bedeuten,

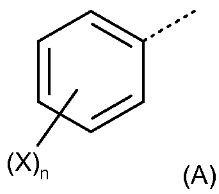 (A) 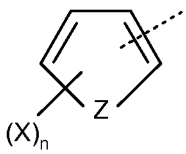 (B) 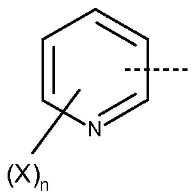 (C)

wobei in den Formeln (A), (B) und (C)

X gleiche oder verschiedene Reste ausgewählt aus der Gruppe bestehend aus $C_{1-18}$-Al-kyl, $C_{1-18}$-Alkoxy und $C_{1-18}$-Dialkylamino, bevorzugt $C_{1-12}$-Alkyl, $C_{1-12}$-Alkoxy und $C_{1-12}$-Dialkylamino, besonders bevorzugt $C_{1-4}$-Alkyl, $C_{1-4}$-Alkoxy und $C_{1-4}$-Dialkylamino, ganz besonders bevorzugt Methyl, Methoxy oder Dimethylamino,

Z Sauerstoff (O), Schwefel (S), oder $NR^9$ (wobei $R^9$ $C_{1-4}$-Alkyl, bevorzugt Methyl oder Ethyl, ganz besonders bevorzugt Methyl bedeutet), bevorzugt Sauerstoff (O) oder Schwefel (S), besonders bevorzugt Sauerstoff (O),

n eine ganze Zahl von 0 bis 3, bevorzugt 0 oder 1 oder 2, besonders bevorzugt 0 oder 1 bedeuten.

Unter einem Alkoxycycloalkyl bzw. einem Alkylcycloalkyl im Sinne der vorliegenden Erfindung ist ein Cycloalkyl-Rest zu verstehen der mindestens eine Alkoxy- oder Alklygruppe aufweist.

Ganz besonders bevorzugt steht $R^1$ für einen Rest der Formel (A) in denen X, Z und n jeweils die o.g. Bedeutungen haben.

Wenn $R^1$ die o.g. Bedeutung hat, dann bedeutet $R^2$ bevorzugt Wasserstoff (H), Methyl, Ethyl oder Phenyl.

Als Carbonylverbindung können auch cyclische Ketone (beispielsweise Cyclohexanon) eingesetzt werden. In solchen Fällen bilden $R^1$ und $R^2$ gemeinsam einen entsprechenden Ring. In diesem Fall bedeuten $R^1$, $R^2$ gemeinsam bevorzugt $-(CH_2)_j-Y-(CH_2)_k-$, wobei

Y Methylen, Sauerstoff (O), Schwefel (S), oder $NR^3$ (wobei $R^3$ $C_{1-4}$-Alkyl, bevorzugt Methyl oder Ethyl, besonders bevorzugt Methyl ist), bevorzugt Methylen, Sauerstoff (O), oder Schwefel (S), besonders bevorzugt Methylen oder Sauerstoff (O), ganz besonders bevorzugt Methylen, und

j und k unabhängig voneinander eine ganze Zahl von 1 bis 4, bevorzugt 1 bis 2, besonders bevorzugt jeweils 2 bedeuten.

$R^2$ bedeutet insbesondere Wasserstoff (H), Methyl, Ethyl oder Phenyl, besonders bevorzugt Wasserstoff (H), Methyl, oder Phenyl, ganz besonders bevorzugt Wasserstoff (H) oder Methyl oder sogar ausschließlich Wasserstoff (H).

Als Carbonylverbindung werden bevorzugt die folgenden Aldehyde und Ketone eingesetzt:

- Aldehyde:
  Benzaldehyd, 2-Methylbenzaldehyd (o-Tolualdehyd), 3-Methylbenzaldehyd (m-Tolualdehyd), 4-Methylbenzaldehyd (p-Tolualdehyd), 2,5-Dimethylbenzaldehyd, 4-Ethylbenzaldehyd, 4-Isopropylbenzaldehyd (Cuminaldehyd), 4-tert.Butylbenzaldehyd, 2-Methoxybenzaldehyd (o-Anisaldehyd), 3-Methoxybenzaldehyd (m-Anisaldehyd), 4-Methoxybenzaldehyd (Anisaldehyd), 2,3-Dimethoxybenzaldehyd, 2,4-Dimethoxybenzaldehyd, 2,5-Dimethoxybenzaldehyd, 3,4-Dimethoxybenzaldehyd (Veratrumaldehyd), 3,5-Dimethoxybenzaldehyd, 2,4,6-Trimethylbenzaldehyd, 2,4,5-Trimethoxybenzaldehyd (Asaronaldehyd), 2,4,6-Trimethoxybenzaldehyd, 3,4,5-Trimethoxybenzaldehyd, 4-Dimethylaminobenzaldehyd, Furan-2-aldehyd, Furan-3-aldehyd.

- Ketone:
  Methylisoamylketon, 3-Octanon, Aceton, Acetophenon, Benzophenon, 2'-Methylacetophenon, 3'-Methylacetophenon, 4'-Methylacetophenon, 2'- Methoxyacetophenon, 3'-Methoxyacetophenon, 4'-Methoxyacetophenon, 2',4'-Dimethylacetophenon, 2',5'-Dimethylacetophenon, 3',4'-Dimethylacetophenon, 3',5'-Dimethylacetophenon, 2',4'-Dimethoxyacetophenon, 2',5'-Dimethoxyacetophenon, 3',4'-Dimethoxyacetophenon, 3',5'-Dimethoxyacetophenon, 2',4',6'- Trimethylacetophenon, 2',4',6'-Trimethoxyacetophenon oder Cyclohexanon.

Demensprechend bevorzugt ist es, dass

$R^1$ und $R^2$ beide Methyl oder beide Phenyl, $R^1$ 3-Methylbutyl und $R^2$ Methyl, oder $R^1$ n-Pentyl und $R^2$ Ethyl bedeuten, oder
$R^1$ ausgewählt ist aus der Gruppe bestehend aus i-Propyl, Phenyl, 2-Methylphenyl, 3-Methylphenyl, 4-Methylphenyl, 2,5-Dimethylphenyl, 4-Ethylphenyl, 4-Isopropylphenyl, 4-tert.-Butylphenyl, 2-Methoxyphenyl, 3-Methoxyphenyl, 4-Methoxyphenyl, 2,3-Dimethoxyphenyl, 2,4-Dimethoxyphenyl, 2,5-Dimethoxyphenyl, 3,4-Dimethoxyphenyl, 3,5-Dimethoxyphenyl, 2,4,5-Trimethylphenyl, 2,4,6-Trimethylphenyl, 2,4,5-Trimethoxyphenyl, 2,4,6-Trimethoxyphenyl, 3,4,5-Trimethoxyphenyl, 2-Dimethylaminophenyl, 3-Dimethylaminophenyl, 4-Dimethylaminophenyl, 2-Furyl, und 3-Furyl, und
$R^2$ Wasserstoff (H) oder Methyl ist;
oder
$R^1$ und $R^2$ gemeinsam n-Pentylen bedeuten.

Besonders bevorzugt ist es, dass

$R^1$ und $R^2$ beide Phenyl bedeuten
oder
$R^1$ ausgewählt ist aus der Gruppe bestehend aus Phenyl, 4-Isopropylphenyl, 4-tert.-Butylphenyl, 4-Methoxyphenyl, 4-Dimethylaminophenyl, 2-Furyl, und 3-Furyl,
$R^2$ Wasserstoff (H) bedeutet,
oder
$R^1$ ausgewählt ist aus der Gruppe bestehend aus Phenyl, 4-Methylphenyl, 4-Methoxyphenyl,
$R^2$ Methyl bedeutet.

Ganz besonders bevorzugt bedeutet $R^1$ Phenyl und $R^2$ Wasserstoff (H).

Soweit nicht explizit etwas Anderes gesagt ist, gilt das im Folgenden für die Reste $R^4$, $R^5$, $R^6$ und $R^7$ gesagte sowohl für Amine der Formel (1) in denen m für 1 steht, als auch für solche in denen m für 2 oder 3 steht.

<u>$R^4$ und $R^5$:</u>

$R^4$ und $R^5$ bedeuten bevorzugt unabhängig voneinander Wasserstoff (H) oder $C_{1-10}$-Alkyl, besonders bevorzugt Wasserstoff (H) oder $C_{1-4}$-Alkyl, insbesondere Methyl. Besonders bevorzugt ist insbesondere der Einsatz von MEOA oder 1-Aminopropan-2-ol (MIPOA) oder dessen Konstitutionsisomer 2-Aminopropan-1-ol (MIPOA') als Aminoalkohol. Entsprechend ganz besonders bevorzugt bedeutet $R^4$ oder $R^5$ Methyl und der jeweils andere Rest Wasserstoff (H) oder $R^4$ und $R^5$ beide Wasserstoff (H).

Das eingesetzte MEOA kann aus der Umsetzung von Ethylenoxid (EO) mit Ammonik erhalten werden. Durch Umsetzung von Propylenoxid (PO) mit Ammoniak lässt sich MIPOA herstellen. Da dies effiziente und mithin kostengünstige Herstellungswege sind, ist es bevorzugt, dass das erfindungsgemäß eingesetzte MEOA und MIPOA durch Umsetzung von EO mit Ammoniak bzw. PO mit Ammoniak hergestellt werden. Das so hergestellte MIPOA enthält üblicherweise geringe Mengen an MIPOA'. Bevorzugt werden MIPOA und MIPOA' nicht voneinander getrennt, sondern ein Gemisch aus MIPOA und MIPOA' in Schritt 1 umgesetzt. Aus der Umsetzung von PO mit Ammoniak erhält man

MIPOA und MIPOA' in einem molaren Verhältnis von 15:1 bis 23:1, bevorzugt 16:1 bis 22:1, besonders bevorzugt 17:1 bis 21:1, ganz besonders bevorzugt von 18:1 bis 20:1. Üblicherweise werden MIPOA und MIPOA' daher in einem entsprechenden molaren Verhältnis, also 15:1 bis 23:1, bevorzugt 16:1 bis 22:1, besonders bevorzugt 17:1 bis 21:1, ganz besonders bevorzugt von 18:1 bis 20:1 eingesetzt.

Wird für m = 1 in Schritt 1 ein solches Gemisch aus MIPOA und MIPOA' mit einer Carbonylverbindung der Formel (3) umgesetzt, so resultieren zwei unterschiedliche Intermediate. Für das aus MIPOA resultierende Intermediat ist $R^4$ = H und $R^5$ = Methyl. Für das aus MIPOA' resultierende Intermediat ist $R^4$ = Methyl und $R^5$ = H. Werden diese beiden Intermediate mit einem Amin der Formel (5) gemäß Schritt 2 umgesetzt erhält man zwei verschiedene Amine der Formel (1). Für das aus MIPOA resultierende Amin ist wiederum $R^4$ = H und $R^5$ = Methyl und für die aus MIPOA' resultierende $R^4$ = Methyl und $R^5$ = H. Das molare Verhältnis, in dem die beiden Amine der Formel (1) gebildet werden, hängt dabei vom molaren Verhältnis von MIPOA zu MIPOA' ab.

Bevorzugt werden mithilfe des erfindungsgemäßen Verfahrens daher insbesondere solche Amine der Formel (1) hergestellt, in der der m für 1 steht und in der $R^4$ Wasserstoff (H) und $R^5$ Methyl (Formel (1.1)) beziehungsweise $R^4$ Methyl und $R^5$ Wasserstoff (H) (Formel (1.2)) bedeuten,

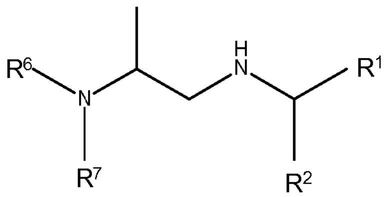 (1.1) 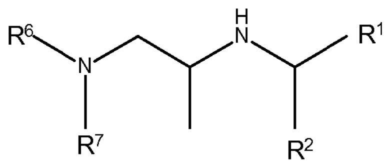 (1.2)

wobei

- gemäß Schritt 1 die Umsetzung von 1-Aminopropan-2-ol (MIPOA) und 2-Aminopropan-1-ol (MIPOA') mit einer Carbonylverbindung der Formel (3) zu den jeweiligen Intermediaten der Formeln (4.1) und (4.2) erfolgt,

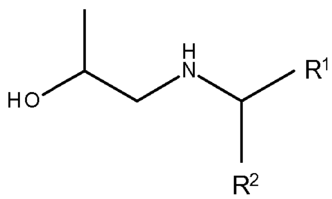 (4.1) 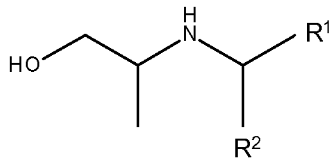 (4.2)

- gemäß Schritt 2 die Umsetzung der in Schritt 1 erhaltenen Intermediate mit einer Aminkomponente der Formel (5) zu den entsprechenden Aminen der Formeln (1.1) und (1.2) erfolgt.

Die Amine der Formel (1.1) und Formel (1.2) werden also gleichzeitig (d.h. nebeneinander hergestellt). Das molare Verhältnis der so erhaltenen Amine der Formel (1.1) und der Formel (1.2) wird im Wesentlichen durch das molare Verhältnis von MIPOA zu MIPOA' bestimmt. Experimentell findet man, dass das molare Verhältnis Amin der Formel (1.1) zu Amin der Formel (1.2) kleiner als das molare Verhältnis von MIPOA zu MIPOA' ist. Angenommen ein Gemisch aus MIPOA und MIPOA' (Molverhältnis 19:1) wird gemäß Schritt 1 mit Benzaldehyd und die resultierenden Intermediate anschließend gemäß Schritt 2 mit Ammoniak umgesetzt. Es resultiert ein Gemisch aus $N^1$-Benzyl-1,2-propandiamin und $N^2$-Benzyl-1,2-propandiamin. Üblicherweise reagieren sekundäre Alkohole schneller zum jeweiligen Amin als primäre Alkohole. In Abhängigkeit von den Reaktionsbedingungen kann das Molverhältnis von $N^1$-Benzyl-1,2-propandiamin zu $N^2$-Benzyl-1,2-propandiamin daher größer als das von MIPOA zu MIPOA' sein.

<u>$R^6$ und $R^7$:</u>

$R^6$ und $R^7$ bedeuten bevorzugt unabhängig voneinander Wasserstoff (H) oder $C_{1-2}$-Alkyl. Besonders bevorzugt ist insbesondere der Einsatz von Dimethylamin (DMA), Methylamin oder Ammoniak. Entsprechend besonders bevorzugt bedeuten $R^6$ und $R^7$ beide Methyl oder beide Wasserstoff (H), oder $R^6$ oder $R^7$ bedeutet Methyl und der jeweils andere Rest Wasserstoff (H). Ganz besonders bevorzugt ist der Einsatz von Ammoniak. Entsprechend ganz besonders bevorzugt bedeuten $R^6$ und $R^7$ für Wasserstoff (H).

Bevorzugt sind mit dem erfindungsgemäßen Verfahren Amine der Formel (1) herstellbar, in der m für 1 steht, wobei

- $R^1$ und $R^2$ beide Methyl oder beide Phenyl, $R^1$ 3-Methylbutyl und $R^2$ Methyl, oder $R^1$ n-Pentyl und $R^2$ Ethyl bedeuten, oder

  $R^1$ ausgewählt ist aus der Gruppe bestehend aus i-Propyl, Phenyl, 2-Methylphenyl, 3-Methylphenyl, 4-Methylphenyl, 2,5-Dimethylphenyl, 4-Ethylphenyl, 4-Isopropylphenyl, 4-tert.-Butylphenyl, 2-Methoxyphenyl, 3-Methoxyphenyl, 4-Methoxyphenyl, 2,3-Dimethoxyphenyl, 2,4-Dimethoxyphenyl, 2,5-Dimethoxyphenyl, 3,4-Dimethoxyphenyl, 3,5-Dimethoxyphenyl, 2,4,5-Trimethylphenyl, 2,4,6-Trimethylphenyl, 2,4,5-Trimethoxyphenyl, 2,4,6-Trimethoxyphenyl, 3,4,5-Trimethoxyphenyl, 2-Dimethylaminophenyl, 3-Dimethylaminophenyl, 4-Dimethylaminophenyl, 2-Furyl, und 3-Furyl, und
  $R^2$ Wasserstoff (H) oder Methyl ist;
  oder
  $R^1$ und $R^2$ gemeinsam n-Pentylen bedeuten,

- $R^4$ oder $R^5$ Methyl und der jeweils andere Rest Wasserstoff (H) bedeuten oder beide Wasserstoff (H) bedeuten,

- $R^6$ und $R^7$ Wasserstoff (H) bedeuten.

Besonders bevorzugt sind mit dem erfindungsgemäßen Verfahren Amine der Formel (1) herstellbar, in der m für 1 steht, wobei

- $R^1$ und $R^2$ beide Phenyl bedeuten
  oder

  $R^1$ ausgewählt ist aus der Gruppe bestehend aus Phenyl, 4-Isopropylphenyl, 4-tert.-Butylphenyl, 4-Methoxyphenyl, 4-Dimethylaminophenyl, 2-Furyl, und 3-Furyl,

  $R^2$ Wasserstoff (H) bedeutet,
  oder

  $R^1$ ausgewählt ist aus der Gruppe bestehend aus Phenyl, 4-Methylphenyl, 4-Methoxyphenyl,

  $R^2$ Methyl bedeutet,

- $R^4$ oder $R^5$ Methyl und der jeweils andere Rest Wasserstoff (H) bedeuten oder beide Wasserstoff (H) bedeuten,

- $R^6$ und $R^7$ Wasserstoff (H) bedeuten.

Ebenfalls besonders bevorzugt sind mit dem erfindungsgemäßen Verfahren Amine der Formel (1) herstellbar, in der m für 1 steht, wobei

- $R^1$ und $R^2$ beide Phenyl bedeuten
  oder

  $R^1$ ausgewählt ist aus der Gruppe bestehend aus Phenyl, 4-Isopropylphenyl, 4-tert.-Butylphenyl, 4-Methoxyphenyl, 4-Dimethylaminophenyl, 2-Furyl, und 3-Furyl,

  $R^2$ Wasserstoff (H) bedeutet,
  oder

  $R^1$ ausgewählt ist aus der Gruppe bestehend aus Phenyl, 4-Methylphenyl, 4-Methoxyphenyl,

  $R^2$ Methyl bedeutet,

- $R^4$ oder $R^5$ Methyl und der jeweils andere Rest Wasserstoff (H) bedeuten,

- $R^6$ und $R^7$ Wasserstoff (H) bedeuten.

Ganz besonders bevorzugt ist die Herstellung von $N^1$-Benzyl-1,2-propylendiamin und $N^2$-Benzyl-1,2-propylen-

diamin, wobei 1-Aminopropan-2-ol (MIPOA) und 2-Aminopropan-1-ol (MIPOA') mit Benzaldehyd gemäß Schritt 1 umgesetzt werden und die resultierenden Zwischenprodukte (N-Benzyl-1-aminopropan-2-ol und N-Benzyl-2-Aminopropan-1-ol) mit Ammoniak gemäß Schritt 2 umgesetzt werden.

Ebenfalls ganz besonders bevorzugt ist die Herstellung von N-Benzyl-ethylendiamin, wobei 2-Aminoethanol mit Benzaldehyd gemäß Schritt 1 umgesetzt wird und das resultierende Zwischenprodukt (N-Benzyl-2-aminoethanol) mit Ammoniak gemäß Schritt 2 umgesetzt wird.

Herstellung von Aminen in denen m für 2 oder 3 steht:

Als Carbonylverbindungen werden entsprechende Dialdehyde und Diketone (m = 2) bzw. Trialdehyde und Triketone (m = 3) eingesetzt. Daneben können aber auch Ketoaldehyde (bspw. 2-Acetylbenzaldehyd, 3-Acetylbenzaldehyd oder 4-Acetylbenzaldehyd) eingesetzt werden.

Bevorzugt ist $R^1$ ein zwei- oder dreiwertiger $C_{4-20}$-, besonders bevorzugt $C_{5-16}$-, besonders bevorzugt $C_{6-14}$-Kohlenwasserstoffrest.

Steht m für 3, so kann $R^1$ die folgende Bedeutung haben:

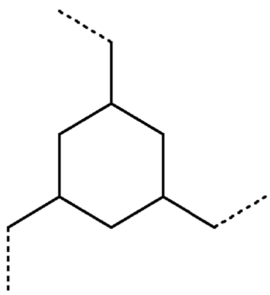

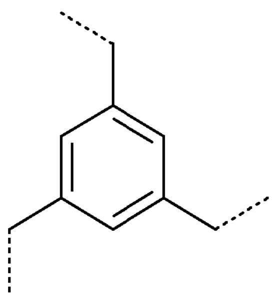

Dabei bedeutet $R^2$ bevorzugt Wasserstoff (H) oder Methyl.

Bevorzugt steht m für 2. Die im Folgenden beschriebenen Reste beziehen sich daher auf die Herstellung von Aminen der Formel (1), in der m für 2 steht.

Bevorzugt steht $R^1$ für einen gegebenenfalls substituierten Phenylen-, Cyclohexylen-, Dicycloheptylen, Tricyclododecylen-, Pentacyclopentadecylen-, Furandiyl, Tetrahydrofurandiyl, Thiophendiyl, Tetrahydrothiophendiyl, oder N,N'-Piperazin-bis(2,2-dimethylpropan)diyl-Rest.

Besonders bevorzugt steht $R^1$ für eine Phenylen-Rest der Formel (D)

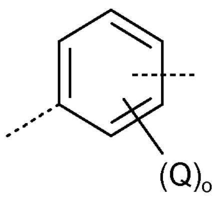 (D)

wobei

o für 0 oder 1 oder 2 steht, und
Q für gleiche oder verschiedene Reste ausgewählt aus der Gruppe bestehend aus Alkyl und Alkoxy mit jeweils 1 bis 4 Kohlenstoffatomen steht.

Weiterhin besonders bevorzugt steht $R^1$ für einen Cyclohexylen-Rest der Formel (E), wobei o und Q die o.g. Bedeutungen aufweisen.

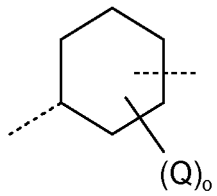

(E)

Weiterhin besonders bevorzugt steht $R^1$ für einen Furan oder Thiophen-Rest der Formel (F), wobei Z für ein Sauerstoff (O) oder Schwefel (S) steht.

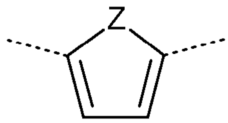

(F)

Weiterhin besonders bevorzugt steht $R^1$ für einen Tetrahydrofuran- oder für einen Tetrahydrothiohen-Rest der Formel (G), wobei Z. die oben genannte Bedeutung aufweist.

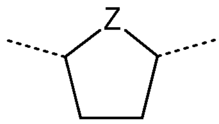

(G)

Bevorzugt ist $R^1$ insbesondere ausgewählt aus der Gruppe bestehen aus 1,2-Phenylen, 1,3-Phenylen, 1,4-Phenylen, 1,2-Cyclohexylen, 1,3-Cyclohexylen, 1,4-Cyclohexylen, 2(3),5(6)-Bicyclo[2.2.1 ]heptylen, 3(4),8(9)-Tricyclo-[5.2.1 .02,6]decylen, 4(5),11(12)-Pentacyclo-[6.5.1 $.1^{3'6}.0^{2'7}.0^{9'13}$]pentadecylen, 6,12-Penta-cyclo[9.2.1 $.1^{5'8}.0^{4'9}.0^{2'10}$]pentadecylen, N,N'-Bis(2,2-dimethyl-1,3-propylen)piperazin, 2,5-Furylen, 2,5-Thiophenylen, 2,5-Tetrahydrophenylen, besonders bevorzugt aus der Gruppe bestehend aus 1,2-Phenylen, 1,3-Phenylen, 1,4-Phenylen, 1,2-Cyclohexylen, 1,3-Cyclohexylen, 1,4-Cyclohexylen.

Bevorzugt ist dabei $R^2$ Wasserstoff (H) oder Methyl.

Bevorzugt sind mit dem erfindungsgemäßen Verfahren Amine der Formel (1) herstellbar, in der m für 2 steht und

- $R^1$ ausgewählt ist aus der Gruppe bestehen aus 1,2-Phenylen, 1,3-Phenylen, 1,4-Phenylen, 1,2-Cyclohexylen, 1,3-Cyclohexylen, 1,4-Cyclohexylen, 2(3),5(6)-Bicyclo[2.2.1 ]heptylen, 3(4),8(9)-Tricyclo- [5.2.1 .02,6]decylen, 4(5),11(12)-Pentacyclo-[6.5.1 $.1^{3'6}.0^{2'7}.0^{9'13}$]pentadecylen, 6,12-Penta-cyclo[9.2.1 $.1^{5'8}.0^{4'9}.0^{2'10}$]pentadecylen, N,N'-Bis(2,2-dimethyl-1,3-propylen)piperazin, 2,5-Furylen, 2,5-Thiophenylen, 2,5-Tetrahydrophenylen,

  $R^2$ Wasserstoff (H) bedeutet,
  oder
  $R^1$ ausgewählt ist aus der Gruppe bestehen aus 1,2-Phenylen, 1,3-Phenylen, 1,4-Phenylen, 1,2-Cyclohexylen, 1,3-Cyclohexylen, 1,4-Cyclohexylen,
  $R^2$ Methyl bedeutet.

- $R^4$ oder $R^5$ Methyl und der jeweils andere Rest Wasserstoff (H) bedeuten,

- $R^6$ und $R^7$ Wasserstoff (H) bedeuten.

Katalysator:

Die nachfolgende Beschreibung hinsichtlich der möglichen Katalysatoren bezieht sich auf die erfindungsgemäße Herstellung aller möglichen Amine der Formel (1). Sofern nichts anders angegeben beziehen sich die nachfolgenden Ausführungen daher sowohl auf Amine bei denen m für 1 steht, als auch auf solche bei denen m für 2 oder 3 steht.

Der im erfindungsgemäßen Verfahren eingesetzte Heterogen-Hydrierkatalysator kann im Folgenden auch als "Katalysator" bezeichnet werden.

Bevorzugt wird im erfindungsgemäßen Verfahren als Heterogen-Hydrierkatalysator in den Schritten 1 und 2

ein Katalysator eingesetzt, der ein oder mehrere Metalle der VIIIB. Gruppe und/oder IB. Gruppe des Periodensystems der Elemente enthält.

Beispiele für solche Elemente sind Cu, Co, Ni und/oder Fe, sowie auch Edelmetalle wie Ru, Pt, Pd, sowie Re. Die Katalysatoren können dotiert sein, etwa mit Ag, Zn, In, Mn, Alkalimetallen (Li, Na, Ka, Rb, Cs) und/oder Mo.

Die Bezeichnung der Gruppen des Periodensystems der Elemente erfolgt gemäß der CAS-Nomenklatur (chemical abstract service).

Bevorzugte Metalle der Gruppe VIIIB des Periodensystems der Elemente sind Fe, Co, Ni, Ru, Rh, Pd, Os, Ir, Pt. Bevorzugte Elemente des Gruppe IB des Periodensystems der Elemente sind Cu, Ag und Au.

Bevorzugt weist der Katalysator ein Trägermaterial auf. Als Trägermaterial werden insbesondere Aluminiumoxid (gamma, delta, theta, alpha, kappa, chi oder Mischungen daraus), Siliziumdioxid, Zirkoniumdioxid (bevorzugt monokline, tetragonale oder kubische Modifikation), Zeolithe, Alumosilicate sowie Mischungen aus diesen Trägern oder Aktivkohle eingesetzt. Besonders bevorzugt ist Zirkoniumdioxid und/oder Aluminiumoxid, ganz besonders bevorzugt Aluminiumoxid, insbesondere gamma-Aluminiumoxid.

Der im erfindungsgemäßen Verfahren eingesetzte Katalysator enthält bevorzugt Cu und/oder Ni und/oder Co, bevorzugt Cu und Ni und/oder Co, besonders bevorzugt Cu und Ni und Co.

Des Weiteren kann der eingesetzte Katalysator auch Cu und/oder Ni und/oder Co und/oder Ru, bevorzugt Cu, Ni, Co und Ru enthalten.

Im erfindungsgemäßen Verfahren werden die Katalysatoren bevorzugt in Form von Katalysatoren eingesetzt, die nur aus katalytisch aktiver Masse und gegebenenfalls einem Verformungshilfsmittel (wie z. B. Graphit oder Stearinsäure), falls der Katalysator als Formkörper eingesetzt wird, bestehen, also keine weiteren katalytisch aktiven Begleitstoffe enthalten.

In diesem Zusammenhang wird das Trägermaterial, insbesondere also Aluminiumoxid ($Al_2O_3$), Zirkoniumdioxid ($ZrO_2$) als zur katalytisch aktiven Masse gehörig gewertet.

Die Katalysatoren werden dergestalt eingesetzt, dass man die katalytisch aktive, zu Pulver vermahlene Masse in das Reaktionsgefäß einbringt oder, dass man die katalytisch aktive Masse nach Mahlung, Vermischung mit Formhilfsmitteln, Formung und Temperung als Katalysatorformkörper - beispielsweise als Tabletten, Kugeln, Ringe, Extrudate (z. B. Stränge) - im Reaktor anordnet.

Die Konzentrationsangaben (in Gew.-%) der Komponenten des Katalysators beziehen sich jeweils - falls nicht anders angegeben - auf die katalytisch aktive Masse des fertigen Katalysators nach dessen letzter Wärmebehandlung und vor dessen Reduktion mit Wasserstoff.

Im erfindungsgemäßen Verfahren wird dementsprechend bevorzugt ein geträgerter kupfer- und nickelhaltiger Katalysator, besonders bevorzugt ein geträgerter kupfer-, nickel- und kobalthaltiger Katalysator eingesetzt.

Ganz besonders bevorzugt wird ein geträgerter kupfer-, nickel- und kobalthaltiger Katalysator eingesetzt, wobei die katalytisch aktive Masse des Katalysators vor dessen Reduktion mit Wasserstoff sauerstoffhaltige Verbindungen des Aluminiums, Kupfers, Nickels und Kobalts und im Bereich von 0,2 bis 0,5 Gew.-%, insbesondere 0,6 bis 3,0 Gew.-%, oder sogar 0,7 bis 2,5 Gew.-% sauerstoffhaltige Verbindungen des Zinns, berechnet als SnO enthält.

Ebenfalls ganz besonders bevorzugt wird ein geträgerter kupfer-, und nickelhaltiger Katalysator eingesetzt, wobei die katalytisch aktive Masse des Katalysators vor dessen Reduktion mit Wasserstoff sauerstoffhaltige Verbindungen des Zirkoniums, Kupfers, und Nickels und im Bereich von 0 bis 5 Gew.-%, besonders 0,1 bis 3 Gew.-%, sauerstoffhaltige Verbindungen des Molybdäns, berechnet als $MoO_3$, enthält.

Die katalytisch aktive Masse des Katalysators, nach dessen letzter Wärmebehandlung und vor dessen Reduktion mit Wasserstoff, ist als die Summe der Massen der katalytisch aktiven Bestandteile und der o. g. Katalysatorträgermaterialien definiert und enthält bevorzugt im Wesentlichen die folgenden Bestandteile: Trägermaterial (bevorzugt also Aluminiumoxid ($Al_2O_3$) und/oder Zirkoniumdioxid ($ZrO_2$)) und sauerstoffhaltige Verbindungen des Kupfers und/oder Nickels und/oder Kobalts sowie gegebenenfalls des Zinns oder Molybdäns.

Die Summe der o. g. Bestandteile der katalytisch aktiven Masse beträgt üblicherweise 70 bis 100 Gew.-%, bevorzugt 80 bis 100 Gew.-%, besonders bevorzugt 90 bis 100 Gew.-%, besonders > 95 Gew.-%, ganz besonders > 98 Gew.-%, insbesondere > 99 Gew.-%, z. B. besonders bevorzugt 100 Gew.-%.

Die katalytisch aktive Masse der erfindungsgemäßen und im erfindungsgemäßen Verfahren eingesetzten Katalysatoren kann weiterhin ein oder mehrere Elemente (Oxidationsstufe 0) oder deren anorganische oder organische Verbindungen, ausgewählt aus den Gruppen I A bis VI A und I B bis VII B und VIII B des Periodensystems, enthalten.

Beispiele für solche Elemente bzw. deren Verbindungen sind:

Übergangsmetalle, wie Mn bzw. $MnO_2$, Mo bzw. $MoO_3$, W bzw. Wolframoxide, Ta bzw. Tantaloxide, Nb bzw. Nioboxide oder Nioboxalat, V bzw. Vanadiumoxide bzw. Vanadylpyrophosphat; Lanthanide, wie Ce bzw. $CeO_2$ oder Pr bzw. $Pr_2O_3$; Erdalkalimetalloxide, wie SrO; Erdalkalimetallcarbonate, wie $MgCO_3$, $CaCO_3$ und $BaCO_3$; Alkalimetalloxide, wie $Na_2O$, $K_2O$; Alkalimetallcarbonate, wie $Li_2CO_3$, $Na_2CO_3$ und $K_2CO_3$; Boroxid ($B_2O_3$).

Bevorzugt enthält die katalytisch aktive Masse des im erfindungsgemäßen Verfahren eingesetzten Katalysators kein Rhenium, kein Ruthenium, kein Eisen und/oder kein Zink, jeweils weder in metallischer (Oxidationsstufe = 0) noch

in einer ionischen (Oxidationsstufe ≠ 0), insbesondere oxidierten, Form.

Bevorzugt enthält die katalytisch aktive Masse des im erfindungsgemäßen Verfahren eingesetzten Katalysators kein Silber und/oder Molybdän, jeweils weder in metallischer (Oxidationsstufe = 0) noch in einer ionischen (Oxidationsstufe ≠ 0), insbesondere oxidierten, Form.

Die Katalysatoren können nach bekannten Verfahren, z. B. durch Fällung, Auffällung, Imprägnierung, hergestellt werden.

Bevorzugte Katalysatoren enthalten in ihrer katalytisch aktiven Masse vor der Behandlung mit Wasserstoff

20 bis 85 Gew.-%, bevorzugt 20 bis 65 Gew.-%, besonders bevorzugt 22 bis 40 Gew.-%, sauerstoffhaltige Verbindungen des Zirkoniums, berechnet als $ZrO_2$,

1 bis 30 Gew.-%, besonders bevorzugt 2 bis 25 Gew.-%, sauerstoffhaltige Verbindungen des Kupfers, berechnet als CuO,

14 bis 70 Gew.-%, bevorzugt 15 bis 50 Gew.-%, besonders bevorzugt 21 bis 45 Gew.-%, sauerstoffhaltige Verbindungen des Nickels, berechnet als NiO, wobei bevorzugt das Molverhältnis von Nickel zu Kupfer größer 1, insbesondere größer 1,2, ganz besonders 1,8 bis 8,5, ist, und

0 bis 5 Gew.-%, besonders 0,1 bis 3 Gew.-%, sauerstoffhaltige Verbindungen des Molybdäns, berechnet als $MoO_3$.

In einer weiteren Variante enthalten diese bevorzugten Katalysatoren zusätzlich in ihrer katalytisch aktiven Masse vor der Behandlung mit Wasserstoff
15 bis 50 Gew.-%, besonders bevorzugt 21 bis 45 Gew.-%, sauerstoffhaltige Verbindungen des Kobalts, berechnet als CoO.

Die sauerstoffhaltigen Verbindungen des Kupfers, Nickels und gegebenenfalls Kobalts, jeweils berechnet als CuO, NiO und CoO, der bevorzugten Katalysatoren sind im Allgemeinen insgesamt in Mengen von 15 bis 80 Gew.-%, bevorzugt 35 bis 80 Gew.-%, besonders bevorzugt 60 bis 78 Gew.-%, in der katalytisch aktiven Masse (vor der Behandlung mit Wasserstoff) enthalten, wobei besonders bevorzugt das Molverhältnis von Nickel zu Kupfer größer 1 ist.

Besonders bevorzugte Katalysatoren enthalten in ihrer katalytisch aktiven Masse vor der Behandlung mit Wasserstoff

20 bis 90 Gew.-%, bevorzugt 40 bis 85 Gew.-%, besonders bevorzugt 60 bis 80 Gew.-%, sauerstoffhaltige Verbindungen des Aluminiums, berechnet als $Al_2O_3$,

1 bis 30 Gew.-%, bevorzugt 2 bis 25 Gew.-%, besonders bevorzugt 3 bis 20 Gew.-%, sauerstoffhaltige Verbindungen des Kupfers, berechnet als CuO,

1 bis 40 Gew.-%, bevorzugt 3 bis 30 Gew.-%, besonders bevorzugt 5 bis 20 Gew.-%, sauerstoffhaltige Verbindungen des Nickels, berechnet als NiO, wobei besonders bevorzugt das Molverhältnis von Nickel zu Kupfer größer 1, bevorzugt größer 1,2, besonders bevorzugt 1,8 bis 8,5, ist, und

1 bis 40 Gew.-%, bevorzugt 3 bis 30 Gew.-%, besonders bevorzugt 5 bis 20 Gew.-%, sauerstoffhaltige Verbindungen des Kobalts, berechnet als CoO.

Die sauerstoffhaltigen Verbindungen des Nickels, Kobalts und Kupfers, jeweils berechnet als NiO, CoO und CuO, sind bevorzugt insgesamt in Mengen von 10 bis 80 Gew.-%, besonders bevorzugt 15 bis 60 Gew.-%, ganz besonders bevorzugt 20 bis 40 Gew.-%, in der katalytisch aktiven Masse (vor der Behandlung mit Wasserstoff) enthalten, wobei besonders bevorzugt das Molverhältnis von Nickel zu Kupfer größer 1 ist.

Die Herstellung der oben beschriebenen Katalysatoren ist beispielsweise in WO 2012/000952 A1 (BASF SE) und WO 2014/184039 A1(BASF SE) beschrieben.

Ganz besonders bevorzugte Katalysatoren enthalten in ihrer katalytisch aktiven Masse vor der Behandlung mit Wasserstoff

15 bis 80 Gew.-%, bevorzugt 30 bis 70 Gew.-%, besonders bevorzugt 35 bis 65 Gew.-%, sauerstoffhaltige Verbindungen des Aluminiums, berechnet als $Al_2O_3$,

1 bis 20 Gew.-%, bevorzugt 2 bis 18 Gew.-%, besonders bevorzugt 5 bis 15 Gew.-%, sauerstoffhaltige Verbindungen des Kupfers, berechnet als CuO,

5 bis 35 Gew.-%, bevorzugt 10 bis 30 Gew.-%, besonders bevorzugt 12 bis 28 Gew.-%, ganz besonders bevorzugt 15 bis 25 Gew.-%, sauerstoffhaltige Verbindungen des Nickels, berechnet als NiO,

5,0 bis 35 Gew.-%, bevorzugt 10 bis 30 Gew.-%, besonders bevorzugt 12 bis 28 Gew.-%, ganz besonders bevorzugt 15 bis 25 Gew.-%, sauerstoffhaltige Verbindungen des Kobalts, berechnet als CoO, und

0,2 bis 5,0 Gew.-%, bevorzugt 0,4 bis 4,0 Gew.-%, besonders bevorzugt 0,6 bis 3,0 Gew.-%, ganz besonders bevorzugt 0,7 bis 2,5 Gew.-%, sauerstoffhaltige Verbindungen des Zinns, berechnet als SnO.

Das Molverhältnis von Nickel zu Kupfer beträgt bevorzugt größer 1, besonders bevorzugt größer 1,2, weiter besonders bevorzugt im Bereich von 1,8 bis 8,5.

Die Herstellung solcher ganz besonders bevorzugter Katalysatoren ist beispielsweise in WO 2011/067199 A1 beschrieben.

Die oben dargestellten Katalysatoren können sowohl in Schritt 1 als auch in Schritt 2 eingesetzt werden. In den Schritten 1 und 2 können sowohl jeweils der gleiche, als auch unterschiedliche Katalysatoren eingesetzt werden.

Es ist ferner möglich, dass

- in Schritt 1

  ein Heterogen-Hydrierkatalysator, bei dem es um einen Schalenkatalysator handelt, eingesetzt wird, der mindestens ein Metall der VIII B. Gruppe des Periodensystems der Elemente als Hydriermetall und zusätzlich einen Promotor auf einem oxidischen Träger umfasst, wobei mindestens 80 % des Metalls der VIII B Gruppe des Periodensystems der Elemente, in einer Schicht zwischen der Oberfläche des Katalysators und einer Eindringtiefe, die maximal 80 % des Radius des Katalysators, gerechnet von der Oberfläche des Katalysators, entspricht, vorliegt,
  oder
  ein Heterogen-Hydrierkatalysator, bei dem es sich um einen geträgerten Katalysator handelt, eingesetzt wird, der als katalytisch aktives Metall Pd und/oder Pt enthält und Aktivkohle oder Aluminiumoxid als Träger aufweist, und

- in Schritt 2
  ein Katalysator eingesetzt wird, der Cu und/oder Ni und/oder Co enthält.

In Schritt 2 kann dabei jeder der oben als bevorzugt, besonders bevorzugt und ganz besonders bevorzugt herausgestellten Katalysatoren, die Cu und/oder Ni und/oder Co enthalten, eingesetzt werden.

Nachfolgend werden der Schalenkatalysator sowie der geträgerte Pd/Pt-Katalysator näher beschrieben. Dabei kann in Schritt 1 jeder der im weiteren Verlauf des Textes als bevorzugt, besonders bevorzugt und ganz besonders bevorzugt herausgestellten Katalysator eingesetzt werden.

Schalenkatalysator:

Bevorzugt liegt das Metall der VIIIB. Gruppe des Periodensystems der Elemente in der definierten Schale im Wesentlichen homogen verteilt vor.

Bevorzugt liegt der Promotor über den gesamten Querschnitt des Katalysators im Wesentlichen homogen verteilt vor.

In einer bevorzugten Ausführungsform weist der Katalysator einen Durchmesser im Bereich von 1,5 bis 10 mm auf, wobei mindestens 80 % des Metalls der VIIIB. Gruppe des Periodensystems der Elemente in einer Schicht zwischen der Oberfläche des Katalysators und einer Eindringtiefe von maximal 1000 µm, gerechnet von der Oberfläche des Katalysators, vorliegt.

In einem solchen Katalysator bildet das Metall der VIIIB. Gruppe des Periodensystems der Elemente eine Schalenstruktur in dem Katalysator aus.

Der erfindungsgemäß einsetzbare Katalysator weist bevorzugt einen Durchmesser im Bereich von 1,5 bis 9 mm auf. In besonders bevorzugten Ausführungsformen beträgt der Durchmesser der erfindungsgemäß einsetzbare Katalysatoren 2,0 bis 5 mm, insbesondere 2,5 bis 3,5 mm.

In dem erfindungsgemäß einsetzbaren Katalysator liegen bevorzugt mindestens 80 %, vorzugsweise mindestens 90 %, besonders bevorzugt mindestens 95 %, insbesondere mindestens 98 %, speziell 100 %, des Metalls der VIIIB. Gruppe des Periodensystems der Elemente in einer Schicht zwischen der Oberfläche des Katalysators und einer Eindringtiefe von maximal 1000 µm, gerechnet von der Oberfläche des Katalysators, vor.

Der erfindungsgemäß einsetzbare Katalysator enthält ein Metall der VIII B Gruppe des Periodensystems der Elemente (Fe, Ru, Os, Co, Rh, Ir, Ni, Pd, Pt). In einer bevorzugten Ausführungsform der vorliegenden Erfindung handelt es sich um Palladium.

Der erfindungsgemäß einsetzbare Katalysator enthält darüber hinaus mindestens einen Promotor. Beispielsweise kann es sich hierbei um weitere Metalle der VIIIB., IB. und IIB. Gruppe des Periodensystems der Elemente handeln (Cu, Ag, Au, Zn, Cd, Hg). In einer bevorzugten Ausführungsform enthalten die erfindungsgemäß einsetzbaren Katalysatoren neben dem Metall der VIIIB. Gruppe des Periodensystems der Elemente noch mindestens ein Metall aus der IB. Gruppe des Periodensystems der Elemente. Besonders bevorzugt ist hierbei Silber.

In einer besonders bevorzugten Ausführungsform enthält der erfindungsgemäß einsetzbare Katalysator Palladium und Silber.

Der erfindungsgemäß einsetzbare Katalysator kann beliebige Formen, beispielsweise Stränge, Hohlstränge, Tabletten, Ringe, sphärische Teilchen oder Kugeln, aufweisen. Bevorzugt ist es, wenn der Katalysator als Strang ausgebildet ist.

Die Metalle können in reiner metallischer Form vorliegen, aber auch in Form von Verbindungen, beispielsweise in Form von Metalloxiden. Unter den Betriebsbedingungen des Schrittes 2 liegen sie im Allgemeinen in Form von Metallen vor. Die Umwandlung etwaiger Oxide in Metalle kann auf dem Fachmann bekannte Weise vor dem Einsatz des Katalysators in einem Hydrierverfahren in oder außerhalb eines Hydrierreaktors, beispielsweise durch Vorreduzierung und, falls für Manipulationen mit dem vorreduzierten Katalysator erforderlich oder vorteilhaft, anschließende oberflächliche Passivierung erfolgen.

Der Gehalt des Katalysators an Metall oder Metallen der VIIIB. Gruppe des Periodensystems, insbesondere Palladium, beträgt vorzugsweise mindestens 0,01 Gew.-%, besonders bevorzugt mindestens 0,1 Gew.-%, insbesondere mindestens 0,15 Gew.-%. Vorzugsweise liegt dieser Gehalt bei höchstens 5 Gew.-%, besonders bevorzugt höchstens 1 Gew.-%, insbesondere höchstens 0,6 Gew.-%. Niedrigere und höhere Gehalte sind zwar möglich, aber im Normalfall wegen zu niedriger Aktivität oder zu hohen Rohstoffkosten wirtschaftlich unbefriedigend. In einer besonders bevorzugten Ausführungsform wird nur ein Hydriermetall, insbesondere Palladium, verwendet.

Das Verhältnis der Mengen von Hydriermetall der VIIIB. Gruppe des Periodensystems der Elemente und Zusatz- oder Dotierstoffen ist ein im Einzelfall zu optimierender Parameter. Vorzugsweise beträgt das Atomverhältnis von Metall der VIIIB. Gruppe des Periodensystems der Elemente, besonders bevorzugt Palladium, zu dem Promotor, besonders bevorzugt Silber, vorzugsweise 0,1 - 10, besonders bevorzugt 2 - 7, insbesondere 2,5 - 6.

Der oxidische Träger des erfindungsgemäß einsetzbaren Katalysators ist bevorzugt Aluminiumoxid, besonders bevorzugt in einer Mischung aus $\delta$-, $\theta$- und $\alpha$-Aluminiumoxid. Der Träger kann neben unvermeidbaren Verunreinigungen in gewissem Umfang auch andere Zusätze enthalten. Beispielsweise können andere anorganische Oxide wie Oxide von Metallen der IIA., IIIB., IVB., IIIA. und IVA. Gruppe des Periodensystems der Elemente enthalten sein, insbesondere Siliziumdioxid, Titandioxid, Zirkondioxid, Zinkoxid, Magnesiumoxid, Natriumoxid und/oder Kalziumoxid. Der maximale Gehalt des Trägers an solchen von Aluminiumoxid verschiedenen Oxiden ist vom tatsächlich vorhandenen Oxid abhängig, aber im Einzelfall anhand des Röntgenbeugungsdiagramms des Hydrierkatalysators zu ermitteln, da eine Änderung der Struktur mit einer signifikanten Änderung des Röntgenbeugungsdiagramms einhergeht. Im Allgemeinen liegt der Gehalt an solchen, von Aluminiumoxid verschiedenen Oxiden, unterhalb von 50 Gew.-%, vorzugsweise unterhalb von 30 Gew.-%, besonders bevorzugt unterhalb von 10 Gew.-%. Der Reinheitsgrad des Aluminiumoxids liegt vorzugsweise höher als 99 %.

Die Herstellung solcher Schalenkatalysatoren, einschließlich ihrer oxidischen Träger ist in der WO 2007/107477 A1 (BASF AG), insbesondere auf den Seiten 5 bis 10 beschrieben.

Pd/Pt-Katalysator:

Erfindungsgemäß bevorzugte Übergangsmetallkatalysatoren sind solche, die als Aktivkomponenten eines oder mehrere Metalle ausgewählt aus der Gruppe der Metalle Pd und Pt enthalten. Besonders bevorzugt ist das Metall Pd als zumindest eine, insbesondere einzige, Aktivkomponente. (Aktivkomponente = katalytisch aktive Komponente).

Trägermaterialien der erfindungsgemäß einzusetzenden Katalysatoren sind bevorzugt Aktivkohle oder Aluminiumoxid, besonders bevorzugt Aktivkohle.

Ein im Rahmen der vorliegenden Erfindung besonders bevorzugter Katalysator ist Pd auf Aktivkohle (Pd/C).

Die genannten Katalysatoren weisen vorteilhafterweise einen Gehalt an Pd und/oder Pt, von 0,1 bis 25 Gew.-%, bevorzugt von 0,5 bis 15 Gew.-% und besonders bevorzugt von 4 bis 11 Gew.-% [jeweils bezogen auf das reduzierte Metall bzw. die reduzierten Metalle (Oxidationsstufe 0) des fertigen Katalysators und bezogen auf das Gesamtgewicht des trockenen Katalysators] auf.

Derartige Katalysatoren sind kommerziell zugänglich und beispielsweise unter den Bezeichnungen Degussa E1002, Degussa E101, Degussa E105, Degussa E106, Engelhard C3630, Heraeus K201, Heraeus K202, Heraeus K203, Heraeus K204, Heraeus K219 erhältlich.

Der Katalysator kann besonders einen Wassergehalt im Bereich von 1 bis 150 Gew.-%, weiter besonders im Bereich von 3 bis 10 Gew.-%, (jeweils bezogen auf das Gewicht des trockenen Katalysators) aufweisen.

Reaktionsführung:

Die nachfolgende Beschreibung hinsichtlich der möglichen Reaktoren, Reaktionsführung, Reaktionsbedingungen, etc. bezieht sich auf die erfindungsgemäße Herstellung aller möglichen Amine der Formel (1). Sofern nichts anders angegeben beziehen sich die nachfolgenden Ausführungen daher sowohl auf Amine bei denen m für 1 steht, als auch auf solche bei denen m für 2 oder 3 steht.

Die Schritte 1 und 2 werden üblicherweise in der Flüssigphase durchgeführt. Gegebenenfalls kann einer Verdünnung der Edukte mit einem geeigneten Lösungsmittel wie Tetrahydrofuran, Dioxan, N-Methylpyrrolidon, Ethylenglykoldimethylether, Toluol oder Xylol erfolgen. In Schritt 1 können zudem Alkohole (bspw. Methanol oder Isopropanol) eingesetzt werden. Je nach den gewählten Reaktionsbedingungen wird ein gewisser Teil der Edukte bzw. des Lösungsmittels entsprechend seinem Partialdruck gasförmig vorliegen.

Das erfindungsgemäße Verfahren lässt sich sowohl kontinuierlich als auch diskontinuierlich durchführen.

Als Reaktoren für die Schritte 1 und 2 kommen beispielsweise Autoklav, Rührkessel oder Festbettreaktoren bspw. Rohrbündel- oder Schachtreaktoren (wobei unter den Festbettreaktoren Schachtreaktoren bevorzugt sind) in Frage.

Schritt 1:

Bei dem in Schritt 1 gebildeten erfindungsgemäßen Reaktionsprodukt handelt es sich üblicherweise um ein Imin bzw. Schiff'sche Base.

In Schritt 1 kann das aus der Umsetzung des Aminoalkohols mit der Carbonylverbindung gebildete Reaktionsprodukt *in situ* zum Intermediat hydriert werden (*in* situ-Reaktionsführung). Hierbei werden der Aminoalkohol und die Carbonylverbindung in Gegenwart von Wasserstoff ($H_2$) und des Heterogen-Hydrierkatalysators umgesetzt. Ebenso ist es möglich, zunächst den Aminoalkohol und die Carbonylverbindung umzusetzen und anschließend das resultierende Reaktionsprodukt (ggf. nach weiterer Aufreinigung) erfindungsgemäß zu hydrieren. Die Umsetzung von Aminoalkohol und Carbonylverbindung erfolgt dabei nicht in Gegenwart von Wasserstoff ($H_2$) und Heterogen-Hydrierkatalysator.

Bei einer *in situ*-Reaktionsführung liegt die Reaktionstemperatur in Schritt 1 bevorzugt bei 20 bis 250 °C, besonders bevorzugt 40 bis 200 °C, ganz besonders bevorzugt 60 bis 180 °C. Schritt 1 des erfindungsgemäßen Verfahrens wird bevorzugt bei einem Absolutdruck (= Reaktionsdruck) von 10 bis 200 bar, besonders bevorzugt 30 bis 150 bar und ganz besonders bevorzugt bei 50 bis 120 bar durchgeführt. Dabei setzt sich der Reaktionsdruck aus den Partialdrücken aller Edukte, ggf. vorhandenem Lösungsmittel, Produkte, etwaiger Nebenprodukte und sonstiger Verunreinigungen zusammen.

Erfolgt die Reaktionsführung nicht *in situ* so erfolgt die Umsetzung von Aminoalkohol und Carbonylverbindung zum erfindungsgemäßen Reaktionsprodukt bevorzugt bei einer Temperatur von 0 bis 250 °C. Der Absolutdruck liegt bevorzugt bei 1 bis 200 bar. Das oben hinsichtlich der Partialdrücke gesagte gilt entsprechend. Die Umsetzung des erfindungsgemäßen Reaktionsproduktes zum Intermediat erfolgt bevorzugt bei den oben für die *in situ*-Reaktionsführung genannten Bereich für Druck und Temperatur.

Schritt 1 wird bevorzugt bei Vollumsatz gefahren. D.h. der Umsatz bezogen auf den Aminoalkohol oder die Carbonylverbindung, je nachdem welche dieser Verbindungen im Unterschuss vorliegt, beträgt ungefähr 100%. Insbesondere beträgt der Umsatz 70 bis 95%, 80 bis 98% oder sogar 90 bis 100%. Im Unterschuss liegt diejenige Verbindung vor, die theoretisch vollständig umgesetzt werden kann (bevorzugt wird der Aminoalkohol im Überschuss und die Carbonylverbindung im Unterschuss eingesetzt; siehe unten). Der Umsatz kann beispielsweise ermittelt werden, indem eine kleine Probemenge aus dem Reaktor entnommen wird und deren Zusammensetzung analysiert wird, zum Beispiel mittels Gaschromatographie. Der Umsatz (U) errechnet sich gemäß folgender Formel:

$$U = 1 - \frac{n}{n_0} * 100\%$$

mit n = Stoffmenge des/der nicht umgesetzten Aminoalkohols/Carbonylverbindung
$n_0$ = Stoffmenge des/der ursprünglich eingesetzten Aminoalkohols/Carbonylverbindung

Der Aminoalkohol der Formel (2) wird bevorzugt in der 0,8 bis 1,9-fachen, besonders bevorzugt 0,9 bis 1,5-fachen und ganz besonders bevorzugt in der 1,1 bis 1,3-fachen oder sogar in der 1,1-fachen bis 1,2-fachen molaren Menge, bezogen auf die Stoffmenge der Carbonylgruppen der Carbonylverbindung der Formel (3), eingesetzt.

Ein besonderer Vorteil ergibt sich, wenn der Aminoalkohl mit einem geringen Überschuss gemäß den o.g. Bereichen (maximal der 1,9-fachen molaren Menge) eingesetzt wird. Es ist dann nicht notwendig, den Aminoalkohl aus dem roh-Intermediat abzutrennen, da er in Schritt 2 nicht stört. Der überschüssige Aminoalkohl kann nach erfolgtem Schritt 2 destillativ entfernt werden. Der so entfernte Aminoalkohl kann gegebenenfalls erneut in Schritt 1 eingesetzt werden (bspw. durch Rückführung im kontinuierlichen Verfahren). Aufgrund des nur geringen Überschusses an Aminoalkohl ist der energetische und apparative Aufwand gering. Werden nur geringe Mengen des Amins hergestellt, so kann der Aminoalkohol nicht beliebig in Schritt 1 zurückgeführt werden, sondern muss stattdessen entsorgt (bspw. verbrannt) werden. Dies stellt eine Verbesserung gegenüber dem Stand der Technik dar, wo üblicherweise 1,2-EDA oder 1,2-PDA mit einem höheren Überschuss als dem 1,9-fachen eingesetzt werden, weil das Verhältnis von zu entsorgendem Edukt zu hergestelltem Produkt im erfindungsgemäßen Verfahren geringer ist. Dieser Effekt ist umso größer, je geringer der Überschuss des eingesetzten Aminoalkohols ist. Insofern eignet sich das erfindungsgemäße Verfahren in dieser Ausführungsform insbesondere zur Herstellung von kleineren Mengen der erfindungsgemäßen Amine. Folglich wird das erfindungsgemäße Verfahren bevorzugt diskontinuierlich betrieben. Ebenso kann es kontinuierlich betrieben werden, wobei die Schritte 1 und 2 jeweils in Festbettreaktoren (demselben oder verschiedenen) durchgeführt werden.

Der Wasserstoff ($H_2$) wird bevorzugt in der 1 bis 10-fachen, besonders bevorzugt in der 2- bis 5-fachen, ganz besonders bevorzugt in der 3- bis 4-fachen molaren Menge bezogen auf die Stoffmenge der Carbonylgruppen der Carbonylverbindung der Formel (3) eingesetzt.

Schritt 1 kann in einem Autoklav oder einem Rührkessel durchgeführt werden. Diese Reaktoren eigenen sich insbesondere für den Satzbetrieb (Batchbetrieb). Bei einem Rührkessel ist zudem ein Semibatchbetrieb (Zuführung eines Edukts oder einer Eduktlösung) möglich.

Im Batchbetrieb werden üblicherweise der Aminoalkohol und die Carbonylverbindung sowie der Heterogen-Hydrierkatalysator (Katalysator), bevorzugt als Suspensionskatalysator, im Reaktor (bspw. Autoklav oder Rührkessel) vorgelegt. Der gewünschte Reaktionsdruck kann beispielsweise durch Aufpressen von Wasserstoff ($H_2$) eingestellt werden. Ebenso ist es möglich, den Aminoalkohol und die Carbonylverbindung in einem geeigneten Reaktor miteinander umzusetzen und anschließend das resultierende Reaktionsprodukt (ggf. nach weiterer Aufarbeitung) in demselben oder einem anderen Reaktor in Gegenwart von Wasserstoff ($H_2$) sowie dem Heterogen-Hydrierkatalysator umzusetzen.

Im Semibatchbetrieb werden üblicherweise der Aminoalkohol sowie der Katalysator, bevorzugt als Suspensionskatalysator, im Rührkessel vorgelegt und anschließend Wasserstoff aufgepresst, bis der gewünschte Reaktionsdruck eingestellt ist. Die Carbonylverbindung wird dann bei Reaktionstemperatur und Reaktionsdruck zugegeben. Die Zugabe kann portionsweise oder kontinuierlich erfolgen. Die Zugabe erfolgt solange bis der gewünschte Umsatz (siehe oben) erreicht ist. Es ist ebenso möglich, die Carbonylverbindung vorzulegen und den Aminoalkohol zuzugeben. Hierbei erfolgt die Hydrierung des Reaktionsproduktes der Umsetzung von Aminoalkohl und Carbonylverbindung *in situ.*

Daneben können der Aminoalkohol und die Carbonylverbindung auch in einem geeigneten Reaktor umgesetzt werden (wobei entweder der Aminoalkohol oder die Carbonylverbindung vorgelegt und die andere Verbindung zugegeben wird) und anschließend das resultierende Reaktionsprodukt (ggf. nach weiterer Aufarbeitung) in demselben oder einem anderen Reaktor in Gegenwart von Wasserstoff ($H_2$) sowie dem Heterogen-Hydrierkatalysator hydriert werden.

Im Batch- und Semibatchbetrieb wir der gewählte Katalysator vorteilhaft in einer solchen Menge eingesetzt, dass die Menge an Katalysator (wasserfrei gerechnet) bezogen auf die Menge an eingesetztem Aminoalkohol der Formel (2) im Bereich von 0,1 bis 30,0 Gew.-%, besonders im Bereich von 1,0 bis 15,0 Gew.-%, beträgt.

Bei der kontinuierlichen Reaktionsführung wird üblicherweise ein Festbettreaktor (siehe oben) eingesetzt. Die Hydrierung des durch Umsetzung von Aminoalkohol und die Carbonylverbindung resultierenden Reaktionsproduktes erfolgt dabei wie oben beschrieben *in situ.* Hierbei beträgt die Katalysatorbelastung üblicherweise 0,01 bis 2, insbesondere 0,1 bis 1 kg Carbonylverbindung der Formel (3) / ($l_{Kat}$ • h), ($l_{Kat}$ = Katalysatorvolumen). Dabei lässt sich mit und ohne Rückführung arbeiten. Bei einer solchen Rückführung wird ein Teil des den Reaktor verlassenden Produktstroms wieder in den Reaktor zurückgeführt. Üblicherweise wird der rückgeführte Produktstrom dabei abgekühlt, um so ein homogeneres Temperaturprofil über die Länge des Reaktors zu erreichen.

Bei einer kontinuierlichen Reaktionsführung können gasförmige Edukte (insbesondere Wasserstoff ($H_2$)) im Kreis geführt werden. Die Kreisgasmenge liegt bevorzugt im Bereich von 40 bis 1500 $m^3$ (bei Betriebsdruck) / [$m^3$ Katalysator (Schüttvolumen) • h], insbesondere im Bereich von 100 bis 700 $m^3$ (bei Betriebsdruck) / [$m^3$ Katalysator (Schüttvolumen) • h]. Das Kreisgas enthält bevorzugt mindestens 10, besonders 50 bis 100, ganz besonders 80 bis 100, Vol.% $H_2$

Die Wahl eines entsprechenden Reaktors sowie der Reaktionsführung hängt im Wesentlichen von der gewünschten Menge des herzustellenden Amins ab. Bei eher kleinen Mengen ist der Einsatz eines Autoklavens oder Rührkessels im Batchbetrieb bevorzugt. Sollen größere Mengen (bspw. mehrere Tonnen) des Amins hergestellt werden, ist der Semibatchbetrieb oder sogar die kontinuierliche Reaktionsführung vorteilhaft.

Das in Schritt 1 gebildete Reaktionswasser kann aus dem gebildeten roh-Intermediat entfernt werden.

Schritt 2:

Im Gegensatz zu Schritt 1 erfolgt in Schritt 2 die Umsetzung des Intermediats mit der Aminkomponente zwangsläufig in Gegenwart von Wasserstoff und einem entsprechenden Katalysator.

Die Reaktionstemperatur in Schritt 2 beträgt bevorzugt 150 bis 250 °C, besonders bevorzugt 170 °C bis 230 °C und ganz besonders bevorzugt 180 bis 220 °C. Schritt 2 des erfindungsgemäßen Verfahrens wird bevorzugt bei einem Absolutdruck (= Reaktionsdruck) von 50 bis 250 bar, besonders bevorzugt 70 bis 220 bar und ganz besonders bevorzugt bei 100 bis 200 bar durchgeführt. Dabei setzt sich der Reaktionsdruck aus den Partialdrücken aller Edukte, ggf. vorhandenem Lösungsmittel, Produkte, etwaiger Nebenprodukte und sonstiger Verunreinigungen zusammen.

Die Aminkomponente der Formel (5) wird bevorzugt in einer 1,5- bis 100-fachen, besonders bevorzugt in einer 1,5- bis 30-fachen, ganz besonders bevorzugt in einer 2- bis 25-fachen oder sogar in einer 2,5 bis 20-fachen molaren Menge, bezogen auf die Stoffmenge der Alkoholgruppen des Intermediats der Formel (4), eingesetzt.

Der Wasserstoff ($H_2$) wird bevorzugt in der 0,1 bis 10-fachen, besonders bevorzugt in der 1- bis 5-fachen, ganz besonders bevorzugt in der 2- bis 4-fachen molaren Menge bezogen auf die Stoffmenge der Alkoholgruppen des Intermediats der Formel (4) eingesetzt.

Es können üblicherweise ungefähr 95% des in Schritt 1 erhaltenen Intermediats umgesetzt werden. Bevorzugt beträgt der Umsatz bezogen auf das Intermediat 20 bis 100%, besonders bevorzugt 50 bis 97% und ganz besonders bevorzugt 70 bis 95%. Der Umsatz (U) errechnet sich gemäß folgender Formel:

$$U = 1 - \frac{n}{n_0} * 100\%$$

mit n = Stoffmenge des nicht umgesetzten Intermediats
no = Stoffmenge des ursprünglich eingesetzten Intermediats

Genau wie bei Schritt 1 hängt die Wahl des Reaktors sowie der Reaktionsführung im Wesentlichen von der gewünschten Menge des herzustellenden Amins der Formel (1) ab.

Wurde das Intermediat im Batchbetrieb hergestellt so erfolgt dessen weitere Umsetzung gemäß Schritt 1 üblicherweise ebenfalls im Batchbetrieb. Dabei kann das in Schritt 1 gebildete Intermediat im Reaktor (bspw. Rührkessel) verbleiben und anschließend (in demselben Rührkessel) gemäß Schritt 2 umgesetzt werden.

Schritt 2 kann bspw. in einem Autoklav oder einem Rührkessel durchgeführt werden. Diese Reaktoren eigenen sich insbesondere für den Satzbetrieb (Batchbetrieb).

Wie Schritt 1 kann auch Schritt 2 kontinuierlich in einem Festbettreaktor durchgeführt werden. Das für Schritt 1 bezüglich möglicher Reaktoren, Rückführung, Kreisgas und Kreisgasmenge gesagte gilt entsprechend für Schritt 2

Im Batchbetrieb wir der gewählte Katalysator vorteilhaft in einer solchen Menge eingesetzt, dass die Menge an Katalysator (wasserfrei gerechnet) bezogen auf die Menge an eingesetztem Intermediat der Formel (4) im Bereich von 0,1 bis 30,0 Gew.-%, besonders im Bereich von 1 bis 25,0 Gew.-%, beträgt.

Bei der kontinuierlichen Reaktionsführung im Festbettreaktor beträgt die Katalysatorbelastung üblicherweise 0,01 bis 2, insbesondere 0,3 bis 0,6 kg Intermediat der Formel (4) / ($I_{Kat}$ • h), ($I_{Kat}$ = Katalysatorvolumen).

Bei der kontinuierlichen Reaktionsführung können die Schritte 1 und 2 in verschiedenen Reaktoren durchgeführt werden, wobei

- der Aminoalkohol, Wasserstoff ($H_2$) und die Carbonylverbindung kontinuierlich in einen ersten Reaktor geführt werden, wo die Umsetzung gemäß Schritt 1 zum Intermediat erfolgt, und
- das den ersten Reaktor verlassende Intermediat, Wasserstoff ($H_2$) und die Aminkomponente kontinuierlich in einen zweiten Reaktor geführt werden, wo die Umsetzung gemäß Schritt 2 zum Amin der Formel (1) erfolgt.

Es ist sowohl möglich, dass dabei beide Reaktoren mit demselben als auch mit verschiedenen Heterogen-Hydrierkatalysatoren ausgestattet sind. Bevorzugt wird das gemäß Schritt 1 hergestellte Intermediat durch eine oder mehrere Druckabscheider (bspw. einem Hochdruck- und ggf. einem Mitteldruckabscheider), ansonsten aber ohne weitere Aufreinigung dem zweiten Reaktor zugeführt und dort gemäß Schritt 2 umgesetzt. Die entsprechenden Reaktionsbedingungen für die Schritte 1 und 2 können wie oben bereits beschrieben gewählt werden. Entsprechendes gilt für die möglichen Reaktoren.

Bevorzugt werden bei einer kontinuierlichen Reaktionsführung die Schritte 1 und 2 nacheinander in demselben Reaktor an demselben Heterogen-Hydrierkatalysator durchgeführt, wobei

- der Aminoalkohol, Wasserstoff ($H_2$) und die Carbonylverbindung kontinuierlich in einen Reaktor geführt werden, wo

die Umsetzung gemäß Schritt 1 zum Intermediat erfolgt,

- das den Reaktor verlassende Intermediat in einem geeigneten Behälter zwischengespeichert wird, und nachdem die gewünschte Menge an Intermediat hergestellt worden ist,
- das Intermediat aus dem Behälter, Wasserstoff ($H_2$) und die Aminkomponente kontinuierlich in den Reaktor geführt werden, wo die Umsetzung gemäß Schritt 2 zum Amin der Formel (1) erfolgt.

Üblicherweise wird zunächst die gewünschte Menge des erfindungsgemäßen Intermediats hergestellt und in dem Behälter (bspw. einem Tank) zwischengespeichert. Üblicherweise wird der Reaktoraustrag dabei durch einen oder mehrere Druckabscheider (bspw. einem Hochdruck- und ggf. einem Mitteldruckabscheider) geleitet. Anschließend wird besagtes Intermediat zusammen mit Wasserstoff und dem Amin der Formel (4) zurück in den Reaktor geleitet. Die entsprechenden Reaktionsbedingungen für die Schritte 1 und 2 können wie oben bereits beschrieben gewählt werden. Entsprechendes gilt für die möglichen Reaktoren.

Daneben ist es ebenfalls möglich, die Schritte 1 und 2 gleichzeitig in demselben Reaktor an demselben Heterogen-Hydrierkatalysator durchzuführen. Hierzu werden alle Edukte gemeinsam in einem Reaktor vorgelegt und dort umgesetzt. Dabei wird das Intermediat *in situ* gebildet und gemäß Schritt 2 zum erfindungsgemäßen Amin umgesetzt. Hierbei ist sowohl eine kontinuierliche als auch eine diskontinuierliche Reaktionsführung möglich. Bevorzugt ist allerdings, das Intermediat nicht *in situ* zu bilden und umzusetzen, d.h. die Schritte 1 und 2 wie oben beschrieben nacheinander durchzuführen.

Das erfindungsgemäß hergestellte roh-Amin der Formel (1) kann weiter gereinigt werden. Beispielsweise kann es einem oder mehreren Destillationsschritten unterworfen werden. Üblicherweise können zunächst überschüssige Aminkomponenten (bspw. Ammoniak), ggf. überschüssiger Aminoalkohol und das in den Schritten 1 und 2 gebildete Reaktionswasser abdestilliert werden. Durch einen nachfolgenden Destillationsschritt lassen sich Hochsieder abtrennen.

Es sei darauf hingewiesen, dass das erfindungsgemäße Verfahren nicht auf solche Ausführungsformen beschränkt ist, in denen genau ein Amin der Formel (1) hergestellt wird.

Die erfindungsgemäßen Amine können mit dem erfindungsgemäßen Verfahren auch gleichzeitig (d.h. nebeneinander) hergestellt werden, beispielsweise indem

- gemäß Schritt 1 mehr als ein Aminoalkohol und/oder mehr als eine Carbonylverbindung umgesetzt werden und die resultierenden Intermediate mit einer oder ggf. mehreren Aminkomponenten umgesetzt werden, oder
- ein gemäß Schritt 1 erhaltenes Intermediat in Schritt 2 mit mehr als einer Aminkomponente umgesetzt wird.

Beispielsweise lässt sich ein Gemisch aus N-Benzyl-1,2-ethandiamin und N-(4-Methoxybenzyl)-1,2-ethandiamin durch Umsetzung von MEOA mit Benzaldehyd und 4-Methoxybenzaldehyd in Schritt 1 und anschließender Umsetzung der resultierenden Intermediate gemäß Schritt 2 mit Ammoniak herstellten. Ebenso kann beispielsweise ein Gemisch von Aminoalkoholen (zum Beispiel MIPOA und MIPOA') eingesetzt werden. Solche Ausführungsformen wurden weiter oben bereits beschrieben.

Wie oben bereits gesagt, können erfindungsgemäß auch Amine in denen m für 2 oder 3 steht, nebeneinander hergestellt werden. Beispielsweise können Terephthalaldehyd und 1-4-Cyclohe-xandicarbaldehyd gemeinsam gemäß Schritt 1 mit MIPOA umgesetzt werden und die resultierenden Zwischenprodukte gemäß Schritt 2 mit Ammoniak umgesetzte werden. Es resultiert ein Gemisch aus 1,4-Bis(2-aminopropylaminomethyl)benzol und 1,4-Bis(2-aminopropylaminome-thyl)cyclohexan, bei denen es sich jeweils um Amine der Formel (1) handelt.

Werden bei der Herstellung von Aminen, in denen m für 2 oder 3 steht, mehrere Aminoalkohole und/oder mehrere Aminkomponenten eingesetzt, so können die Molekülfragmente $[R^6R^7NCHR^5CHR^4NHCHR^2]$- in der Formel (1) voneinander verschieden sein. Entsprechendes gilt für die Molekülfragmente im Intermediat der Formel (4). Wird beispielsweise Terephthalaldehyd gleichzeitig mit MIPOA und MIPOA' und die resultierenden Intermediate anschließend mit Ammoniak umgesetzt, so entstehen neben 1,2-Bis(2-aminopropylaminomethyl)benzol und 1,2-Bis(1-amino-2-methylethylaminomethyl)benzol auch 1,2-(2-aminopropylaminomethyl)(2-amino-1-methylethylaminomethyl)benzol. Bei der letztgenannten Verbindung bedeuten $R^4$ Wasserstoff(H) und $R^5$ Methyl in einem Molekülfragment und in dem anderen Molekülfragment $R^4$ Methyl und $R^5$ Wasserstoff (H).

Je nach gewünschtem Verwendungszweck können die in den vorangehenden Absätzen beschriebenen Gemische direkt eingesetzt werden. Ebenso ist es möglich, die jeweiligen Amine (beispielsweise via Destillation) zu trennen.

Um jeden Zweifel auszuschließen, sei darauf hingewiesen, dass die entsprechenden oben genannten bevorzugten, besonders bevorzugten und ganz besonders bevorzugten molaren Verhältnisse sich, für den Fall, dass mehrere Amine der Formel (1) gleichzeitig hergestellt werden, also mehrere Edukte eingesetzt werden, auf die Summe der Stoffmengen der jeweiligen Edukte beziehen. Dies bedeutet insbesondere Folgendes. Der/die Aminoalkohol/e der Formel (2) wird/werden in den o.g. molaren Mengen bezogen auf die Stoffmenge der Carbonylgruppen der Carbonylverbindung/en der Formel (3) eingesetzt. Wasserstoff ($H_2$) wird in den o.g. molaren Mengen bezogen auf die Stoffmenge der Carbonylgruppen der Carbonylverbindung/en der Formel (3) eingesetzt. Die Aminkomponente/n der Formel (5)

werden in den o.g. molaren Mengen bezogen auf die Stoffmenge der Alkoholgruppen des/der Intermediates/Intermediate der Formel (4) eingesetzt. Wasserstoff ($H_2$) wird bevorzugt in den o.g. molaren Mengen bezogen auf die Stoffmenge der Alkoholgruppen des/ der Intermediates/Intermediate der Formel (4) eingesetzt. Sollen beispielsweise 0,5 Mol Benzaldehyd und 0,5 Mol 2-Methylbenzaldehyd mit der 1,3-fachen molaren Menge von MIPOA und MIPOA' (molares Verhältnis 19:1) umgesetzt werden, so müssen 1,235 Mol MIPOA und 0,065 Mol MIPOA' eingesetzt werden.

Die folgenden Beispiele dienen der Verdeutlichung der Erfindung, ohne sie in irgendeiner Weise zu beschränken.

BEISPIELE

Gaschromatographie (für erfindungsgemäße Beispiele): RTX5 Amin Säule 30m Länge, 0,32mm Durchmesser, 1,5μm Schichtdicke. Temperaturprogramm: 60 °C Starttemperatur, mit 4°C/min auf 280°C, 15 min. halten.

Erfindungsgemäß:

Schritt 1:

In einen Glaskolben mit Stickstoffüberlagerung, Thermometer und einem Rückflusskühler wurden 82,6 g (1,1 Mol) Monoisopropanolamin (MIPOA und MIPOA' im molaren Verhältnis 95:5, im Folgenden gemeinsam als "Monoisopropanolamin" bezeichnet) gegeben, der Kolben wurde in ein Eisbad getaucht und dann innerhalb von 30 min. Benzaldehyd (106,1 g, 1,0 Mol) zugetropft. Das Gemisch erwärmte sich aufgrund freiwerdender Reaktionswärme. Nach Abklingen der Reaktionswärme erstarrte das Gemisch aus Schiffscher Base und überschüssigem Monoisopropanolamin. Nun wurde so viel THF zugegeben, dass das Gemisch bei 35°C eine klare Lösung bildete. Der gesamte Reaktionsansatz wurde auf vier Hochdruckautoklaven von jeweils 160 mL Volumen, mit einem Schrägblattrührer, einem Katalysatorkorb, einer Wasserstoffzuleitung und Thermoelement aufgeteilt. Jeder Autoklav besaß eine Elektroheizung. Jeder Katalysatorkorb wurde mit 6 g eines Pd/Ag-Katalysator auf Aluminiumoxidträger (0,3% Pd, 0,1% Ag, Stränge, 2,8 mm Durchmesser gemäß WO 2007/107477 A1 (BASF AG), Beispiel auf Seite 19 f.) befüllt. Die Autoklaven wurden verschlossen und inertisiert, 20 bar Wasserstoff aufgepresst, und dann der Inhalt auf 100°C erwärmt. Bei Erreichen der Reaktionstemperatur wurde der Wasserstoffdruck auf 80 bar erhöht und 12 h gerührt. Verbrauchter Wasserstoff wurde nachgepresst. Eine Probe wurde mittels Gaschromatographie analysiert.

Das Analysenergebnis mit Retentionszeit, Zuordnung und GC-Flächenprozent ist in Tabelle 1 dargestellt. THF ist herausgerechnet. Die Konzentration des THFs war etwa 63%. Die Zuordnung erfolgte mit Gaschromatographie mit Massenspektrometrie-Kopplung (GC-MS). Die Selektivität zu N-Benzyl-monoisopropanolamin (N-Benzyl-MIPOA und N-Benzyl-MIPOA') betrug 95,6%.

Tabelle 1: Analysenergebnisse der Benzylierung von Monoisopropanolamin

| Retentionszeit | 6,13 | 6,32 | 7,63 | 14,88 | 26,09 | 26,38 | |
|---|---|---|---|---|---|---|---|
| Verbindung | Monoisopropanolamin | Unbekannt | Toluol | Benzyl amin | N-Benzyl-MIPOA | N-Benzyl-MIPOA' | Summe Sonstige |
| GC-Fl-% | 2,19 | 0,49 | 1,00 | 0,04 | 91,55 | 4,02 | 0,71 |

Schritt 2:

Das erhaltene Gemisch wurde mit Triethylenglykoldimethylether (TEGDME) versetzt, um in der nachfolgenden Reaktion die Bildung von volatilen Verbindungen oder Hochsiedern beurteilen zu können. Ein GC der eingesetzten Reaktionsmischung enthielt 7,5% TEGDME (GC-Fl.-%). 50 g des Gemischs (ca. 0,19 mol N-Benzyl-monoisopropanolamin) wurde dann in einen 0,3 L Hochdruckautoklaven mit Scheiben-Begasungsrührer, Thermoelement, Elektroheizung, Wasserstoffzuleitung und Flüssigzuleitung für Ammoniak gegeben, in den bereits 10 g eines reduziert-passivierten Aminierungskatalysators (enthaltend Ni, Co, Cu, Sn auf $Al_2O_3$ und erhalten gemäß WO 2011/067199 A1 (BASF AG), Beispiel 5) in einem Katalysatorkorb eingebaut worden waren. Anschließend wurden 65 g (3,8 mol) Ammoniak zugegeben, entsprechend einem Molverhältnis von etwa 20:1. Nun wurde bei Raumtemperatur 30 bar $H_2$ aufgepresst und unter Rühren auf 180°C erhitzt. Bei Erreichen dieser Temperatur wurde der Druck durch Nachpressen von Wasserstoff auf 200 bar erhöht. Bei Druckabfall auf 190 bar wurde mit Wasserstoff auf 200 bar nachgepresst (Wasserstoffverbrauch durch Reduktion des Katalysators). Nun wurde 10 h unter diesen Bedingungen gerührt. Dann wurde das Reaktionsgemisch filtriert, um den Katalysator abzutrennen.

Das Gemisch wurde mit derselben Methode gaschromatographisch analysiert wie oben angegeben. THF und TEGDME wurden herausgerechnet. TEGDME wurde mit 7,5% detektiert, entsprechend der relativen Menge in der Einsatzstoffmischung. Daraus wurde geschlossen, dass weder volatile Nebenprodukte noch Hochsieder, die nicht GC-gängig sind, gebildet wurden. Die Ergebnisse sind in Tabelle 2 aufgeführt.

Tabelle 2: Analysenergebnisse der Aminierung von N-Benzyl-monoisopropanolamin

| Retentionszeit | 6,13 | 7,63 | 14,88 | 25,88 | 26,09 | 26,19 | 26,38 | |
|---|---|---|---|---|---|---|---|---|
| Verbindung | 1,2-PDA | Toluol | Benzyl amin | $N^1$-Benzyl-PDA | N-Benzyl-MIPOA | N2-Benzyl-PDA | N-Benzyl-MIPOA’ | Summe Sonstige |
| GC-FI-% | 5,8 | 1,2 | 9,3 | 72,6 | 4,4 | 2,0 | 2,0 | 2,7 |

Zur Berechnung der Selektivität wird GC-FI-% mit Gew.-% gleichgesetzt.

Der molare Umsatz von N-Benzyl-monoisopropanolamin betrug 93%, die Selektivität zur Summe der beiden Isomeren $N^1$-Benzyl-PDA und $N^2$-Benzyl-PDA des Zielproduktes N-Benzylpropandiamin (N-Benzyl-PDA) 78%. Als weitere Wertprodukte wurden 1,2-Propandiamin und Benzylamin gebildet. Unter Berücksichtigung der Molmassen der identifizierten Produkte, die aus Benzaldehyd gebildet wurden, betrug die molare Selektivität der Umsetzung von Benzaldehyd zu N-Benzyl-PDA ($N^1$-Benzylpropandiamin und $N^2$-Benzylpropandiamin) 77%. Unter Berücksichtigung des Teilumsatzes des N-Benzyl-Monoisopropanolamin (Intermediat) zu N-Benzyl-PDA war die Selektivität sogar 83%.

Vergleichsbeispiel (nicht erfindungsgemäß):

6,2 g (0,08 mol) 1,2-Propylendiamin (1,2-PDA) wurden mit 35,6 g Isopropanol in einen Glaskolben mit Rückflusskühler mit Stickstoffüberdeckung, Thermometer und Rührer gegeben und dazu wurden 8,9 g (0,08 mol) Benzaldehyd getropft. Das Ganze wurde 2h gerührt und dann in einen Autoklaven überführt, der einen Katalysatorkorb befüllt mit 20 ML (10,7 g) des oben beschriebenen Palladium-Katalysators (gemäß WO 2007/107477 A1 (BASF AG), Beispiel auf Seite 19 f.) enthielt. Bei Raumtemperatur wurden 20 bar $H_2$ aufgepresst und gerührt. Dann wurde auf 90°C erhitzt und bei Erreichen dieser Temperatur der Wasserstoffdruck auf 90 bar erhöht und 4 h bei diesen Bedingungen gerührt. Der Wasserstoffdruck wurde annähernd konstant gehalten, indem verbrauchter Wasserstoff nachgepresst wurde. Anschließend wurde eine Probe wurde mittels Gaschromatographie analysiert. Die Methode war die folgende: Säule DB1, Länge 30m, Durchmesser 0,32mm; Schichtdicke 3$\mu$m; Temperaturprogramm 80°C Starttemperatur, 10°C/min auf 280°C, 50 min. halten bei 280°C.

Das Analysenergebnis mit Retentionszeit, Zuordnung und GC-Gewichtsprozent ist in Tabelle 3 dargestellt. Isopropanol ist herausgerechnet.

Tabelle 3: Analsenerebnisse der reduktiven Aminierung von Benzaldehyd mit 1,2-PDA

| Retentionszeit | 5,85 | 7,18 | 18,76 | 18,97 | 26,19 | |
|---|---|---|---|---|---|---|
| Verbindung | 1,2-PDA | Toluol | $N^1$-Benzyl-PDA | $N^2$-Benzyl-PDA | N,N’-Dibenzyl-PDA | Summe Sonstige |
| GC-FI-% | 9,49 | 4,94 | 20,17 | 9,96 | 53,12 | 2,3 |

Der Umsatz von Benzaldehyd betrug 100%, der von 1,2-PDA 90,5%. Unter Berücksichtigung der Molmassen lässt sich die Selektivität der Umsetzung von Benzaldehyd zum N-Benzylpropandiamin berechnen. Diese betrug 25%.

Diskussion der Ergebnisse:

Die Beispiele zeigen, dass mit dem erfindungsgemäßen Verfahren eine deutlich höhere Selektivität (Selektivität von 77 %) erzielen lässt, als durch die Aminierung von Benzaldehyd mit 1,2-PDA (Selektivität von 25 %). Insbesondere überraschend für den Fachmann ist, dass in Schritt 2 keine nennenswerte Bildung von Toluol erfolgt ist. Dem Fachmann ist bekannt, dass im Bereich der Organischen Chemie eine Benzylgruppe als sogenannte Schutzgruppe einer Aminogruppe eingesetzt wird. D.h. die Benzylgruppe, in ihrer Funktion als Schutzgruppe, verhindert, dass die Aminogruppe während einer komplizierten mehrstufigen chemischen Synthese keine unerwünschte Reaktion eingeht. Nach Abschluss der Synthese wird die Schutzgruppe wieder abgespalten. Dies geschieht üblicherweise unter den Reaktionsbedingungen des Schrittes 2. sowie unter Verwendung der eingesetzten Heterogen-Hydrierkatalysatoren. Folglich hätte der Fachmann erwartet, dass in Schritt 2 in solch erheblichem Maße Toluol gebildet wird, also die Aminogruppe wieder entschützt wird, dass N-Benzylpropandiamin nur in sehr geringen Mengen entsteht.

**Patentansprüche**

**1.** Verfahren zur Herstellung von Aminen der Formel (1),

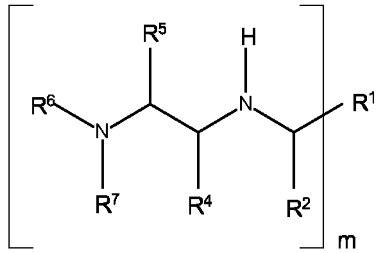

(1)

in der

m für 1, 2 oder 3 steht, wobei

wenn m für 1 steht

$R^1$ aliphatischer $C_{1-60}$-Kohlenwasserstoffrest oder $C_{4-60}$-Kohlenwasserstoffrest, der mindestens einen cycloaliphatischen oder aromatischen Ring enthält, bedeutet, wobei ein solcher $C_{1-60}$- oder $C_{4-60}$-Kohlenwasserstoffrest gegebenenfalls ein oder mehrere Heteroatome, unabhängig voneinander ausgewählt aus der Gruppe bestehend aus Stickstoff (N), Sauerstoff (0), und Schwefel (S) enthalten kann, und
$R^2$ Wasserstoff (H), $C_{1-6}$-Alkyl oder Phenyl bedeutet,
oder
$R^1$, $R^2$ gemeinsam $-(CH_2)_j-Y-(CH_2)_k-$ bedeuten, wobei Y Methylen, Sauerstoff (0), Schwefel (S), oder $NR^3$ (wobei $R^3$ $C_{1-4}$-Alkyl ist) bedeutet und j und k unabhängig voneinander eine ganze Zahl von 1 bis 4 bedeuten,

wenn m für 2 oder 3 steht

$R^1$ zwei- oder dreiwertiger $C_{4-20}$-Kohlenwasserstoffrest bedeutet, der mindestens einen cycloaliphatischen oder aromatischen Ring enthält, wobei ein solcher Kohlenwasserstoffrest gegebenenfalls ein oder mehrere Heteroatome, unabhängig voneinander ausgewählt aus der Gruppe bestehend aus Stickstoff (N), Sauerstoff (0), und Schwefel (S) enthalten kann, und
$R^2$ Wasserstoff (H), $C_{1-6}$-Alkyl oder Phenyl bedeutet, und

$R^4$ und $R^5$ unabhängig voneinander Wasserstoff (H) oder $C_{1-16}$-Alkyl bedeuten,
$R^6$ und $R^7$ unabhängig voneinander Wasserstoff (H), oder $C_{1-4}$-Alkyl bedeuten, umfassend die Schritte

1. Umsetzung eines Aminoalkohols der Formel (2)

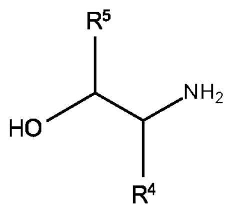

(2)

mit einer Carbonylverbindung der Formel (3)

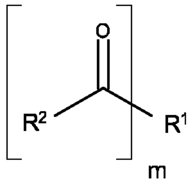

(3)

und anschließender Hydrierung des resultierenden Reaktionsproduktes mit Wasserstoff ($H_2$) an einem Heterogen-Hydrierkatalysator zu einem Intermediat der Formel (4)

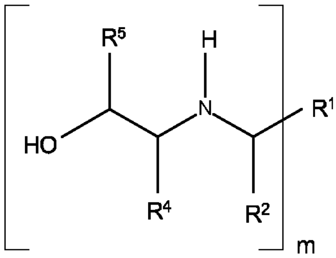

(4)

2. Umsetzung des in Schritt 1 erhaltenen Intermediats mit einer Aminkomponente der Formel (5)

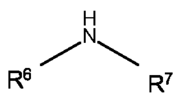

(5)

in Gegenwart von Wasserstoff ($H_2$) und eines Heterogen-Hydrierkatalysators zu einem entsprechenden Amin der Formel (1),
wobei m sowie die Reste $R^1$, $R^2$, $R^4$, $R^5$, $R^6$ und $R^7$ in den Formeln (2) bis (5) dieselbe Bedeutung wie in Formel (1) haben.

**2.** Verfahren nach Anspruch 1, **dadurch gekennzeichnet, dass** in Schritt 1 der Aminoalkohol der Formel (2) in der 0,8 bis 1,9-fachen molaren Menge, bezogen auf die Stoffmenge der Carbonylgruppen der Carbonylverbindung der Formel (3), eingesetzt wird.

**3.** Verfahren nach einem der vorhergehenden Ansprüche, **dadurch gekennzeichnet, dass** in Schritt 2 die Aminkomponente der Formel (5) in der 1,5- bis 10- fachen Menge, bezogen auf die Stoffemenge der Alkoholgruppen des Intermediats der Formel (4), eingesetzt wird.

**4.** Verfahren nach einem der vorhergehenden Ansprüche, zur Herstellung von Aminen der Formel (1) in der m für 2 steht, wobei

$R^1$ für einen gegebenenfalls substituierten Phenylen-, Cyclohexylen-, Dicycloheptylen, Tricyclododecylen-, Pentacyclopentadecylen-, Furandiyl, Tetrahydrofurandiyl, Thiophendiyl, Tetrahydrothiophendiyl, oder N,N'-Piperazin-bis(2,2-dimethylpropan)diyl-Rest steht, und
$R^2$ Wasserstoff ($H_2$), Methyl oder Phenyl ist.

**5.** Verfahren nach einem der vorhergehenden Ansprüche zur Herstellung von Aminen der Formel (1) in der m für 1 steht, wobei

$R^1$ $C_{3-50}$-Alkyl, $C_{3-50}$-Alkoxyalkyl, $C_{4-12}$-Cycloalkyl, $C_{4-30}$-Alkoxycycloalkyl, $C_{4-30}$-Alkylcycloalkyl, oder Rest der Formel (A), (B), oder (C) bedeutet,

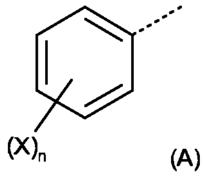 (A) 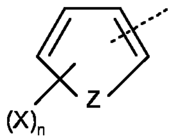 (B) 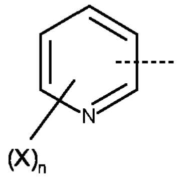 (C)

wobei in den Formeln (A), (B) und (C)

X gleiche oder verschiedene Reste ausgewählt aus der Gruppe bestehend aus $C_{1-18}$-Alkyl, $C_{1-18}$-Alkoxy und $C_{1-18}$-Dialkylamino,
Z Sauerstoff (0), Schwefel (S), oder $NR^9$ (wobei $R^9$ $C_{1-4}$-Alkyl bedeutet),
n eine ganze Zahl von 0 bis 3, bedeuten, und

$R^2$ Wasserstoff (H), Methyl, Ethyl oder Phenyl bedeutet,
oder
$R^1$ und $R^2$ gemeinsam $-(CH_2)_j-Y-(CH_2)_k-$ bedeuten, wobei Y Methylen oder Sauerstoff (0) bedeutet und j und k unabhängig voneinander eine ganze Zahl von 1 bis 2 bedeuten.

**6.** Verfahren nach einem der vorhergehenden Ansprüche zur Herstellung von Aminen der Formel (1) in der m für 1 steht, wobei

$R^1$ und $R^2$ beide Methyl oder beide Phenyl, $R^1$ 3-Methylbutyl und $R^2$ Methyl, oder $R^1$ n-Pentyl und $R^2$ Ethyl bedeuten,
oder
$R^1$ ausgewählt ist aus der Gruppe bestehend aus i-Propyl, Phenyl, 2-Methylphenyl, 3-Methylphenyl, 4-Methylphenyl, 2,5-Dimethylphenyl, 4-Ethylphenyl, 4-Isopropylphenyl, 4-tert.-Butylphenyl, 2-Methoxyphenyl, 3-Methoxyphenyl, 4-Methoxyphenyl, 2,3-Dimethoxyphenyl, 2,4-Dimethoxyphenyl, 2,5-Dimethoxyphenyl, 3,4-Dimethoxyphenyl, 3,5-Dimethoxyphenyl, 2,4,5-Trimethylphenyl, 2,4,6-Trimethylphenyl, 2,4,5-Trimethoxyphenyl, 2,4,6-Trimethoxyphenyl, 3,4,5-Trimethoxyphenyl, 2-Dimethylaminophenyl, 3-Dimethylaminophenyl, 4-Dimethylaminophenyl, 2-Furyl, und 3-Furyl, und
$R^2$ Wasserstoff (H) oder Methyl ist;
oder
$R^1$ und $R^2$ gemeinsam n-Pentylen bedeuten.

**7.** Verfahren nach einem der vorhergehenden Ansprüche zur Herstellung von Aminen der Formel (1) in der m für 1 steht, wobei $R^1$ Phenyl und $R^2$ Wasserstoff bedeutet.

**8.** Verfahren nach einem der vorhergehenden Ansprüche, **dadurch gekennzeichnet, dass** $R^6$ und $R^7$ Wasserstoff (H) bedeuten.

**9.** Verfahren nach einem der vorhergehenden Ansprüche, **dadurch gekennzeichnet, dass** $R^4$ oder $R^5$ Methyl und der jeweils andere Reste Wasserstoff (H) bedeuten oder dass $R^4$ und $R^5$ beide Wasserstoff (H) bedeuten.

**10.** Verfahren nach einem der vorhergehenden Ansprüche zur Herstellung von Aminen der Formel (1), in der m für 1 steht und in der $R^4$ Wasserstoff (H) und $R^5$ Methyl (Formel (1.1)) beziehungsweise $R^4$ Methyl und $R^5$ Wasserstoff (H) (Formel (1.2)) bedeuten,

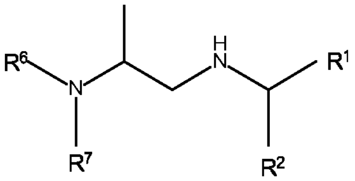 (1.1) 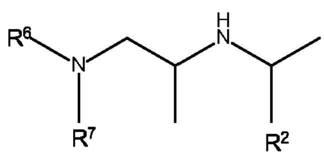 (1.2)

wobei

- gemäß Schritt 1 die Umsetzung von 1-Aminopropan-2-ol (MIPOA) und 2-Aminopro-pan-1-ol (MIPOA') mit einer Carbonylverbindung der Formel (3) zu den jeweiligen Intermediaten der Formeln (4.1) und (4.2) erfolgt,

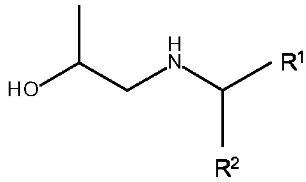
(4.1)

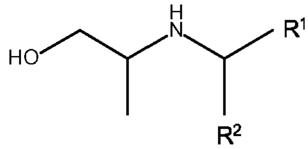
(4.2)

- gemäß Schritt 2 die Umsetzung der in Schritt 1 erhaltenen Intermediate mit einer Aminkomponente der Formel (5) zu den entsprechenden Aminen der Formeln (1.1) und (1.2) erfolgt.

**11.** Verfahren nach dem vorhergehenden Anspruch zur Herstellung von $N^1$-Benzyl-1,2-propylendiamin und $N^2$-Benzyl-1,2-propylendiamin, wobei 1-Aminopropan-2-ol (MIPOA) und 2-Aminopropan-1-ol (MIPOA') mit Benzaldehyd gemäß Schritt 1 umgesetzt werden und die resultierenden Intermediate (N-Benzyl-1-aminopropan-2-ol und N-Benzyl-2-Aminopropan-1-ol) mit Ammoniak gemäß Schritt 2 umgesetzt werden.

**12.** Verfahren nach einem der beiden vorhergehenden Ansprüche, **dadurch gekennzeichnet, dass** 1-Aminopropan-2-ol (MIPOA) und 2-Aminopropan-1-ol (MIPOA') in einem molaren Verhältnis von MIPOA zu MIPOA' von 15:1 bis 23:1 eingesetzt werden.

**13.** Verfahren nach einem der vorhergehenden Ansprüche, **dadurch gekennzeichnet, dass** als Heterogen-Hydrierkatalysator in den Schritten 1 und 2, ein Katalysator eingesetzt wird, der ein oder mehrere Metalle der VIII B. Gruppe und/oder IB. Gruppe des Periodensystems der Elemente enthält.

**14.** Verfahren nach dem vorhergehenden Anspruch, **dadurch gekennzeichnet, dass** der Katalysator ein Trägermaterial aufweist.

**15.** Verfahren nach einem der beiden vorhergehenden Ansprüche, **dadurch gekennzeichnet, dass** als Heterogen-Hydrierkatalysator ein Katalysator eingesetzt wird, der Cu und/oder Ni und/oder Co enthält.

**16.** Verfahren nach dem vorhergehenden Anspruch, **dadurch gekennzeichnet, dass** als Heterogen-Hydrierkatalysator ein geträgerter kupfer-, nickel- und kobalthaltiger Katalysator eingesetzt wird, wobei die katalytisch aktive Masse des Katalysators vor dessen Reduktion mit Wasserstoff sauerstoffhaltige Verbindungen des Aluminiums, Kupfers, Nickels und Kobalts und im Bereich von 0,2 bis 0,5 Gew.-% sauerstoffhaltige Verbindungen des Zinns, berechnet als SnO enthält.

**17.** Verfahren nach Anspruch 13, **dadurch gekennzeichnet, dass**

- in Schritt 1

ein Heterogen-Hydrierkatalysator, bei dem es um einen Schalenkatalysator handelt, eingesetzt wird, der mindestens ein Metall der VIII B Gruppe des Periodensystems der Elemente als Hydriermetall und zusätzlich einen Promotor auf einem oxidischen Träger umfasst, wobei mindestens 80 % des Metalls der VIII B Gruppe des Periodensystems der Elemente, in einer Schicht zwischen der Oberfläche des Katalysators und einer Eindringtiefe, die maximal 80 % des Radius des Katalysators, gerechnet von der Oberfläche des Katalysators, entspricht, vorliegt,
oder
ein Heterogen-Hydrierkatalysator, bei dem es sich um einen geträgerten Katalysator handelt, eingesetzt wird, der als katalytisch aktives Metall Pd und/oder Pt enthält und Aktivkohle oder Aluminiumoxid als Träger aufweist,

- in Schritt 2 ein Katalysator gemäß einem der Ansprüche 13 bis 16 eingesetzt wird.

## Claims

**1.** A process for preparing amines of the formula (1),

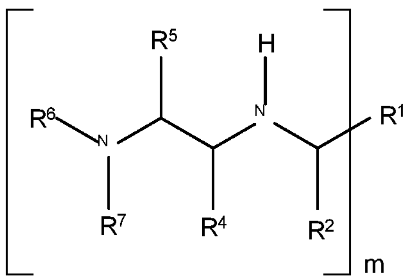

(1)

in which

m is 1, 2 or 3, where

when m is 1

$R^1$ is an aliphatic $C_{1-60}$ hydrocarbon radical or $C_{4-60}$ hydrocarbon radical that contains at least one cycloaliphatic or aromatic ring, said $C_{1-60}$ or $C_{4-60}$ hydrocarbon radical optionally containing one or more heteroatoms independently selected from the group consisting of nitrogen (N), oxygen (O), and sulfur (S), and
$R^2$ is hydrogen (H), $C_{1-6}$ alkyl or phenyl,
or
$R^1$, $R^2$ together are $-(CH_2)_j-Y-(CH_2)_k-$, where Y is methylene, oxygen (O), sulfur (S), or $NR^3$ (where $R^3$ is $C_{1-4}$ alkyl) and j and k are independently an integer from 1 to 4,

when m is 2 or 3

$R^1$ is a di- or trivalent $C_{4-20}$ hydrocarbon radical that contains at least one cycloaliphatic or aromatic ring, said hydrocarbon radical optionally containing one or more heteroatoms independently selected from the group consisting of nitrogen (N), oxygen (O),and sulfur (S), and
$R^2$ is hydrogen (H), $C_{1-6}$ alkyl or phenyl, and

$R^4$ and $R^5$ are independently hydrogen (H) or $C_{1-16}$ alkyl,
$R^6$ and $R^7$ are independently hydrogen (H) or $C_{1-4}$ alkyl, comprising the steps of

1. reacting an amino alcohol of the formula (2)

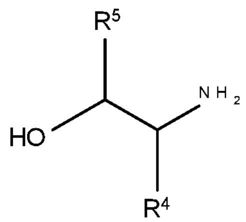

(2)

with a carbonyl compound of the formula (3)

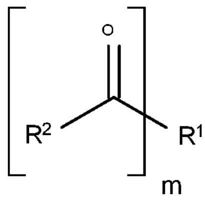

(3)

and subsequent hydrogenation of the resulting reaction product with hydrogen ($H_2$) on a heterogeneous hydrogenation catalyst to form an intermediate of the formula (4)

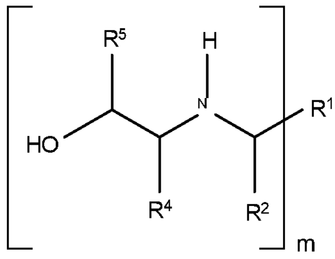

(4)

2. reacting the intermediate obtained in step 1 with an amine component of the formula (5)

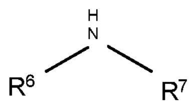

(5)

in the presence of hydrogen ($H_2$) and a heterogeneous hydrogenation catalyst to form a corresponding amine of the formula (1),
where m and the radicals $R^1$, $R^2$, $R^4$, $R^5$, $R^6$, and $R^7$ in the formulas (2) to (5) are as defined in formula (1) .

**2.** The process according to claim 1, **characterized in that**, in step 1, the amino alcohol of the formula (2) is used in an amount that is 0.8 to 1.9 times the molar amount based on the molar amount of the carbonyl groups in the carbonyl compound of the formula (3).

**3.** The process according to either of the preceding claims, **characterized in that**, in step 2, the amine component of the formula (5) is used in an amount that is 1.5 to 10 times the amount based on the molar amount of the alcohol groups in the intermediate of the formula (4).

**4.** The process according to any of the preceding claims, for preparing amines of the formula (1) in which m is 2, where

$R^1$ is an optionally substituted phenylene, cyclohexylene, dicycloheptylene, tricyclododecylene, pentacyclopentadecylene, furandiyl, tetrahydrofurandiyl, thiophenediyl, tetrahydrothiophenediyl, or N,N'-piperazine-bis(2,2-dimethylpropane)diyl radical, and
$R^2$ is hydrogen ($H_2$), methyl or phenyl.

**5.** The process according to any of the preceding claims for preparing amines of the formula (1) in which m is 1, where

$R^1$ is $C_{3-50}$ alkyl, $C_{3-50}$ alkoxyalkyl, $C_{4-12}$ cycloalkyl, $C_{4-30}$ alkoxycycloalkyl, $C_{4-30}$ alkylcycloalkyl, or radical of the formula (A), (B) or (C),

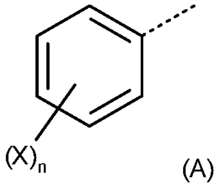 (A) 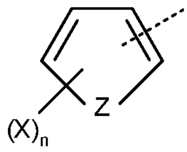 (B) 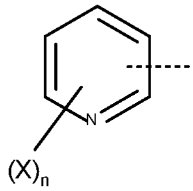 (C)

where, in formulas (A), (B), and (C)

X are identical or different radicals selected from the group consisting of $C_{1-18}$ alkyl, $C_{1-18}$ alkoxy, and $C_{1-18}$ dialkylamino,
Z is oxygen (O), sulfur (S) or $NR^9$ (where $R^9$ is $C_{1-4}$ alkyl),
n is an integer from 0 to 3,

and
$R^2$ is hydrogen (H), methyl, ethyl or phenyl, or
$R^1$ and $R^2$ together are $-(CH_2)_j-Y-(CH_2)_k-$, where Y is methylene or oxygen (O) and j and k are independently an integer from 1 to 2.

**6.** The process according to any of the preceding claims for preparing amines of the formula (1) in which m is 1, where

$R^1$ and $R^2$ are both methyl or both phenyl, $R^1$ is 3-methylbutyl and $R^2$ is methyl, or $R^1$ is n-pentyl and $R^2$ is ethyl, or
$R^1$ is selected from the group consisting of i-propyl, phenyl, 2-methylphenyl, 3-methylphenyl, 4-methylphenyl, 2,5-dimethylphenyl, 4-ethylphenyl, 4-isopropylphenyl, 4-tert-butylphenyl, 2-methoxyphenyl, 3-methoxyphenyl, 4-methoxyphenyl, 2,3-dimethoxyphenyl, 2,4-dimethoxyphenyl, 2,5-dimethoxyphenyl, 3,4-dimethoxyphenyl, 3,5-dimethoxyphenyl, 2,4,5-trimethylphenyl, 2,4,6-trimethylphenyl, 2,4,5-trimethoxyphenyl, 2,4,6-trimethoxyphenyl, 3,4,5-trimethoxyphenyl, 2-dimethylaminophenyl, 3-dimethylaminophenyl, 4-dimethylaminophenyl, 2-furyl, and 3-furyl, and
$R^2$ is hydrogen (H) or methyl;
or
$R^1$ and $R^2$ are together n-pentylene.

**7.** The process according to any of the preceding claims for preparing amines of the formula (1) in which m is 1, where $R^1$ is phenyl, and $R^2$ is hydrogen.

**8.** The process according to any of the preceding claims, **characterized in that** $R^6$ and $R^7$ are hydrogen (H).

**9.** The process according to any of the preceding claims, **characterized in that** $R^4$ or $R^5$ is methyl and the other radical is in each case hydrogen (H) or **in that** $R^4$ and $R^5$ are both hydrogen (H).

**10.** The process according to any of the preceding claims for preparing amines of the formula (1) in which m is 1 and in which $R^4$ is hydrogen (H) and $R^5$ is methyl (formula (1.1)) or $R^4$ is methyl and $R^5$ is hydrogen (H) (formula (1.2))

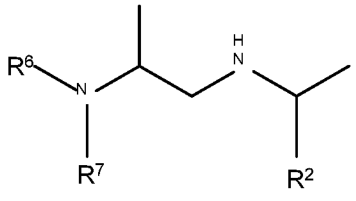 (1.1) 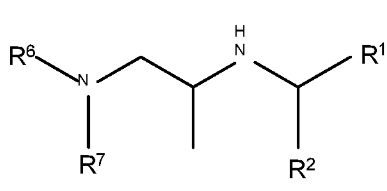 (1.2)

where

- in step 1, 1-aminopropan-2-ol (MIPOA) and 2-ami-nopropan-1-ol (MIPOA') are reacted with a carbonyl compound of the formula (3) to form the respective intermediates of the formulas (4.1) and (4.2),

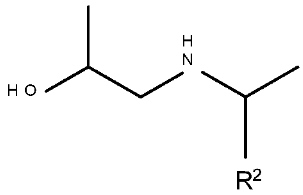 (4.1) 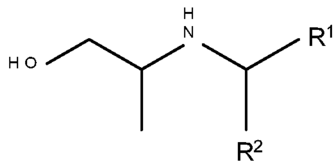 (4.2)

- in step 2, the intermediates obtained in step 1 are reacted with an amine component of the formula (5) to form the corresponding amines of the formulas (1.1) and (1.2).

**11.** The process according to the preceding claim for preparing $N^1$-benzyl-1,2-propylenediamine and $N^2$-benzyl-1,2-propylenediamine, where 1-aminopropan-2-ol (MIPOA) and 2-aminopropan-1-ol (MIPOA') are reacted with benzaldehyde in step 1 and the resulting intermediates (N-benzyl-1-aminopropan-2-ol and N-benzyl-2-aminopropan-1-ol) are reacted with ammonia in step 2.

**12.** The process according to either of the two preceding claims, **characterized in that** 1-aminopropan-2-ol (MIPOA) and 2-aminopropan-1-ol (MIPOA') are used in a molar ratio of MIPOA to MIPOA' of 15:1 to 23:1.

**13.** The process according to any of the preceding claims, **characterized in that** the heterogeneous hydrogenation catalyst used in steps 1 and 2 is a catalyst comprising one or more metals from group VIII B and/or group IB of the periodic table of the elements.

**14.** The process according to the preceding claim, **characterized in that** the catalyst has a support material.

**15.** The process according to either of the two preceding claims, **characterized in that** the heterogeneous hydrogenation catalyst used is a catalyst that comprises Cu and/or Ni and/or Co.

**16.** The process according to the preceding claim, **characterized in that** the heterogeneous hydrogenation catalyst used is a supported catalyst comprising copper, nickel, and cobalt, where the catalytically active mass of the catalyst, prior to the reduction thereof with hydrogen, comprises oxygen-containing compounds of aluminum, copper, nickel, and cobalt and from 0.2% to 0.5% by weight of oxygen-containing compounds of tin, calculated as SnO.

**17.** The process according to claim 13, **characterized in that**

- in step 1

a heterogeneous hydrogenation catalyst that is an eggshell catalyst is used, which comprises at least one metal from group VIII B of the periodic table of the elements as the hydrogenation metal and additionally a promoter on an oxidic support, at least 80% of the metal from group VIII B of the periodic table of the elements being present in a layer between the surface of the catalyst and a penetration depth corresponding to not more than 80% of the radius of the catalyst, calculated from the surface of the catalyst,
or
a heterogeneous hydrogenation catalyst that is a supported catalyst is used, which comprises Pd and/or Pt as the catalytically active metal and has activated carbon or aluminum oxide as support,

- in step 2 a catalyst according to any of claims 13 to 16 is used.

## Revendications

**1.** Procédé pour la préparation d'amines de formule (1),

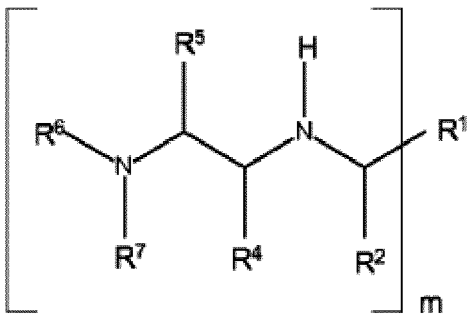

(1)

dans laquelle

m représente 1, 2 ou 3, dans lequel

lorsque m représente 1

$R^1$ signifie un radical aliphatique hydrocarboné en $C_{1-60}$ ou hydrocarboné en $C_{4-60}$, qui contient au moins un cycle cycloaliphatique ou aromatique, un tel radical hydrocarboné en $C_{1-60}$ ou en $C_{4-60}$ pouvant éventuellement contenir un ou plusieurs hétéroatomes, choisis indépendamment les uns des autres dans le groupe constitué par azote (N), oxygène (O), et soufre (S), et
$R^2$ signifie hydrogène (H), $C_{1-6}$-alkyle ou phényle,
ou
$R^1$, $R^2$ signifient ensemble $-(CH_2)_j-Y-(CH_2)_k-$, Y signifiant méthylène, oxygène (O), soufre (S), ou $NR^3$ ($R^3$ étant $C_{1-4}$-alkyle) et j et k signifiant indépendamment l'un de l'autre un nombre entier de 1 à 4,

lorsque m représente 2 ou 3

$R^1$ signifie un radical hydrocarboné divalent ou trivalent en $C_{4-20}$ qui contient au moins un cycle cycloaliphatique ou aromatique, un tel radical hydrocarboné pouvant éventuellement contenir un ou plusieurs hétéroatomes, choisis indépendamment les uns des autres dans le groupe constitué par azote (N), oxygène (O), et soufre (S), et
$R^2$ signifie hydrogène (H), $C_{1-6}$-alkyle ou phényle, et

$R^4$ et $R^5$ signifient indépendamment l'un de l'autre hydrogène (H) ou $C_{1-16}$-alkyle,
$R^6$ et $R^7$ signifient indépendamment l'un de l'autre hydrogène (H) ou $C_{1-4}$-alkyle,
comprenant les étapes de :

1) transformation d'un aminoalcool de formule (2)

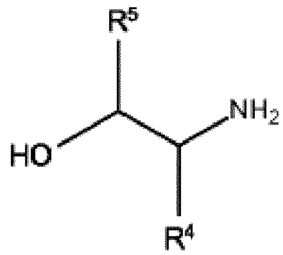

(2)

avec un composé carbonylé de formule (3)

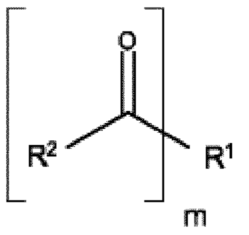

(3)

suivie par l'hydrogénation du produit de réaction résultant avec de l'hydrogène ($H_2$) sur un catalyseur d'hydrogénation hétérogène pour donner un intermédiaire de formule (4)

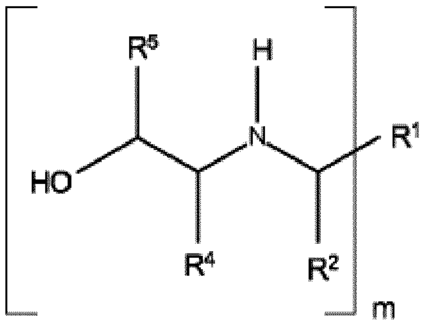

(4)

2) transformation de l'intermédiaire obtenu dans l'étape 1 avec un composant de type amine de formule (5)

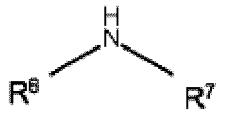

(5)

en présence d'hydrogène ($H_2$) et d'un catalyseur d'hydrogénation hétérogène pour donner une amine correspondante de formule (1),
m ainsi que les radicaux $R^1$, $R^2$, $R^4$, $R^5$, $R^6$ et $R^7$ dans les formules (2) à (5) possédant la même signification que dans la formule (1).

**2.** Procédé selon la revendication 1, **caractérisé en ce que** dans l'étape 1, l'aminoalcool de formule (2) est utilisé en une quantité molaire de 0,8 à 1,9 fois la quantité de matière des groupes carbonyle du composé carbonylé de formule (3).

**3.** Procédé selon l'une quelconque des revendications précédentes, **caractérisé en ce que** dans l'étape 2, le composant de type amine de formule (5) est utilisé en la quantité de 1,5 à 10 fois par rapport à la quantité de matière des groupes alcool de l'intermédiaire de formule (4).

**4.** Procédé selon l'une quelconque des revendications précédentes, pour la préparation d'amines de formule (1) dans laquelle m représente 2,
$R^1$ représentant un radical phénylène, un radical cyclohexylène, un radical dicycloheptylène, un radical tricyclododécylène, un radical pentacyclopentadécylène, un radical furannediyle, un radical tétrahydrofurannediyle, un radical thiophènediyle, un radical tétrahydrothiophènediyle ou un radical N,N'-pipérazine-bis(2,2-diméthylpropane)diyle, éventuellement substitué, et $R^2$ étant hydrogène ($H_2$), méthyle ou phényle.

**5.** Procédé selon l'une quelconque des revendications précédentes pour la préparation d'amines de formule (1) dans laquelle m représente 1,

$R^1$ signifiant $C_{3-50}$-alkyle, $C_{3-50}$-alcoxyalkyle, $C_{4-12}$-cycloalkyle, $C_{4-30}$-alcoxycycloalkyle, $C_{4-30}$-alkylcycloalkyle, ou un radical de formule (A), (B) ou (C),

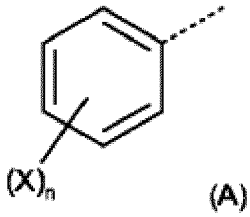

(A)

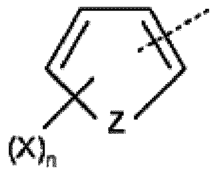

(B)

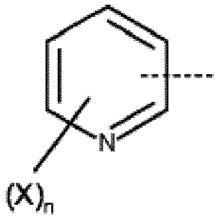

(C)

dans les formules (A), (B) et (C),
X signifiant les radicaux identiques ou différents choisis dans le groupe constitué par $C_{1-18}$-alkyle, $C_{1-18}$-alcoxy et $C_{1-18}$-dialkylamino,
Z signifiant oxygène (O), soufre (S), ou $NR^9$ ($R^9$ signifiant $C_1$
n signifiant un nombre entier de 0 à 3, et
$R^2$ signifiant hydrogène (H), méthyle, éthyle ou phényle,
ou
$R^1$ et $R^2$ signifiant ensemble $-(CH_2)_j-Y-(CH_2)_k-$, Y signifiant méthylène ou oxygène (O) et j et k signifiant indépendamment l'un de l'autre un nombre entier de 1 à 2.

**6.** Procédé selon l'une quelconque des revendications précédentes pour la préparation d'amines de formule (1) dans laquelle m représente 1,

$R^1$ et $R^2$ signifiant tous les deux méthyle ou tous les deux phényle, $R^1$ signifiant 3-méthylbutyle et $R^2$ signifiant méthyle, ou $R^1$ signifiant n-pentyle et $R^2$ signifiant éthyle,
ou
$R^1$ étant choisi dans le groupe constitué par i-propyle, phényle, 2-méthylphényle, 3-méthylphényle, 4-méthylphényle, 2,5-diméthylphényle, 4-éthylphényle, 4-isopropylphényle, 4-tert-butylphényle, 2-methoxyphényle, 3-methoxyphényle, 4-methoxyphényle, 2,3-diméthoxyphényle, 2,4-diméthoxyphényle, 2,5-diméthoxyphényle, 3,4-diméthoxyphényle, 3,5-diméthoxyphényle, 2,4,5-triméthylphényle, 2,4,6-triméthylphényle, 2,4,5-triméthoxyphényle, 2,4,6-triméthoxyphényle, 3,4,5-triméthoxyphényle, 2-diméthylaminophényle, 3-diméthylaminophényle, 4-diméthylaminophényle, 2-furyle et 3-furyle, et
$R^2$ étant hydrogène (H) ou méthyle ;
ou
$R^1$ et $R^2$ signifiant ensemble n-pentylène.

**7.** Procédé selon l'une quelconque des revendications précédentes pour la préparation d'amines de formule (1) dans laquelle m représente 1, $R^1$ signifiant phényle et $R^2$ signifiant hydrogène.

**8.** Procédé selon l'une quelconque des revendications précédentes, **caractérisé en ce que** $R^6$ et $R^7$ signifient hydrogène (H).

**9.** Procédé selon l'une quelconque des revendications précédentes, **caractérisé en ce que** $R^4$ ou $R^5$ signifie méthyle et l'autre radical respectif signifie hydrogène (H) ou **en ce que** $R^4$ et $R^5$ signifient tous les deux hydrogène (H).

**10.** Procédé selon l'une quelconque des revendications précédentes pour la préparation d'amines de formule (1) dans laquelle m représente 1 et dans laquelle $R^4$ signifie hydrogène (H) et $R^5$ signifie méthyle (formule (1.1)), respectivement $R^4$ signifie méthyle et $R^5$ signifie hydrogène (H) (formule (1.2)),

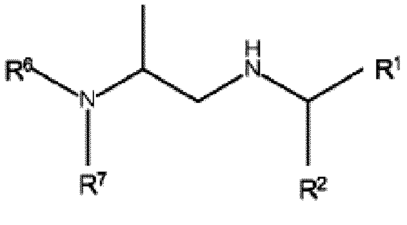

(1.1)

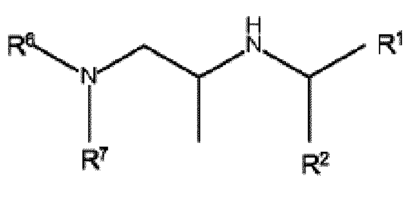

(1.2)

dans lequel

- selon l'étape 1, la transformation de 1-aminopropan-2-ol (MIPOA) et de 2-aminopropan-1-ol (MIPOA') avec un composé carbonylé de formule (3) est réalisée, pour donner les intermédiaires respectifs des formules (4.1) et (4.2),

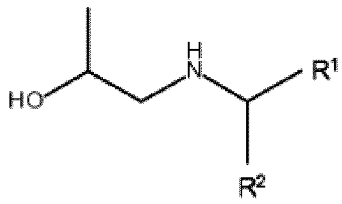 (4.1) 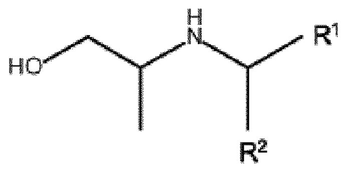 (4.2)

- selon l'étape 2, la transformation des intermédiaires obtenus dans l'étape 1 avec un composant de type amine de formule (5) est réalisée pour donner les amines correspondantes des formules (1.1) et (1.2).

**11.** Procédé selon la revendication précédente pour la préparation de $N^1$-benzyl-1,2-propylènediamine et de $N^2$-benzyl-1,2-propylènediamine, du 1-aminopropan-2-ol (MIPOA) et du 2-aminopropan-1-ol (MIPOA') étant transformés avec du benzaldéhyde selon l'étape 1 et les intermédiaires résultants (N-benzyl-1-aminopropan-2-ol et N-benzyl-2-aminopropan-1-ol) étant transformés avec de l'ammoniac selon l'étape 2.

**12.** Procédé selon l'une des deux revendications précédentes, **caractérisé en ce que** le 1-aminopropan-2-ol (MIPOA) et le 2-aminopropan-1-ol (MIPOA') sont utilisés en un rapport molaire de MIPOA sur MIPOA' de 15 : 1 à 23 : 1.

**13.** Procédé selon l'une quelconque des revendications précédentes, **caractérisé en ce qu'**un catalyseur qui contient un ou plusieurs métaux du groupe VIII B. et/ou du groupe IB. du système périodique des éléments est utilisé en tant que catalyseur d'hydrogénation hétérogène dans les étapes 1 et 2.

**14.** Procédé selon la revendication précédente, **caractérisé en ce que** le catalyseur présente un matériau de support.

**15.** Procédé selon l'une des deux revendications précédentes, **caractérisé en ce qu'**un catalyseur qui contient du Cu et/ou du Ni et/ou du Co est utilisé en tant que catalyseur d'hydrogénation hétérogène.

**16.** Procédé selon la revendication précédente, **caractérisé en ce qu'**un catalyseur supporté contenant du cuivre, du nickel et du cobalt est utilisé en tant que catalyseur d'hydrogénation hétérogène, la masse catalytiquement active du catalyseur, avant sa réduction avec de l'hydrogène, contenant des composés de l'aluminium, du cuivre, du nickel et du cobalt, contenant de l'oxygène et dans la plage de 0,2 à 0,5 % en poids de composés de l'étain contenant de l'oxygène, calculé comme SnO.

**17.** Procédé selon la revendication 13, **caractérisé en ce que**

- dans l'étape 1, un catalyseur d'hydrogénation hétérogène est utilisé, qui est un catalyseur en coque, qui comprend au moins un métal du groupe VIII B du système périodique des éléments en tant que métal d'hydrogénation et de plus un promoteur sur un support d'oxyde, au moins 80 % du métal du groupe VIII B du système périodique des éléments étant présent dans une couche entre la surface du catalyseur et une profondeur de pénétration qui correspond au maximum à 80 % du rayon du catalyseur, calculé à partir de la surface du catalyseur,
ou
un catalyseur d'hydrogénation hétérogène est utilisé, qui est un catalyseur supporté, qui contient, en tant que métal catalytiquement actif, du Pd et/ou du Pt et présente du charbon actif ou de l'oxyde d'aluminium en tant que support,
- dans l'étape 2, un catalyseur selon l'une quelconque des revendications 13 à 16 est utilisé.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 8552078 B2
- WO 2016023839 A1
- WO 2017037069 A1
- WO 2016023837 A1
- WO 2017037070 A1
- WO 2012000952 A1
- WO 2014184039 A1
- WO 2011067199 A1
- WO 2007107477 A1


### In der Beschreibung aufgeführte Nicht-Patentliteratur


- **MOKROV G. V. et al.** *Russian Chemical Bulletin,* vol. 59 (6), 1254-1266
- **KURGANOV A. et al.** *Liebigs Ann. Chem.,* 1980, 786-790